# EUROPEAN PATENT SPECIFICATION

(11) **EP 0 525 123 B1**
(45) Date of publication and mention of the grant of the patent: **17.09.1997**
(21) Application number: 91911326.6
(22) Date of filing: 18.06.1991
(51) Int. Cl.: C07D 311/22, C07D 405/12, C07D 413/12, C07D 417/12, A61K 31/35, A61K 31/535

(54) **ANTIATHEROSCLEROTIC AND ANTITHROMBOTIC 1-BENZOPYRAN-4-ONES AND 2-AMINO-1,3-BENZOXAZINE-4-ONES**
ANTIATHEROSKLEROTISCHE UND ANTITHROMBOTISCHE 1-BENZOPYRAN-4-ON- UND 2-AMINO-1,3-BENZOXAZIN-4-ON-DERIVATE
1-BENZOPYRAN-4-ONES ET 2-AMINO-1,3-BENZOXAZINE-4-ONES ANTIATHERO SCLEROTIQUES ET ANTITHROMBOTIQUES

(30) Priority: 20.06.1990 US 541126
(43) Date of publication of application: 03.02.1993
(73) Proprietor: PHARMACIA & UPJOHN COMPANY, Kalamazoo, Michigan 49001 (US)
(72) Inventor: GAMMILL, Ronald, B., Kalamazoo, MI 49002 (US); JUDGE, Thomas, M., Kalamazoo, MI 49001 (US); MORRIS, Joel, Kalamazoo, MI 49008 (US)
(74) Representative: Perry, Robert Edward
(86) International application number: US9104140
(87) International publication number: WO9119707

(56) References cited:
- EP-A- 0 248 420
- WO-A-90/06921
- US-A- 4 092 416
- Chemical Abstracts, vol. 87, no. 15, 10 October 1977, Columbus, Ohio, US, abstract no. 117750g
- Journal of Heterocyclic Chemistry, vol. 18, no. 4, June 1981, J.R. Bantick et al., pp. 678-684
- European Journal of Medicinal Chemistry, vol. 23, no. 3, 1988, Paris, FR, M. Mazzei et al., pp. 237-242

## Description

### BACKGROUND OF THE INVENTION

The present specification provides methods for use of pharmacologically active substances. Further the present specification provides novel compositions of matter and novel methods of their preparation.

Atherosclerosis in mammals is a disease characterized by the deposition of atherosclerotic plaque on arterial walls. While atherosclerosis exhibits many varied forms and consequences, typical consequences of atherosclerotic diseases include angina pectoris, myocardial infarction, stroke and transient cerebral ischemic attacks. Other forms of atherosclerotic diseases include certain peripheral vascular diseases and other ischemias (e.g., bowel and renal).

Medical science now recognizes that certain forms of atherosclerosis may be preventable or reversible. Agents capable of preventing or reversing atherosclerosis are characterized as exhibiting antiatherosclerotic activity. Since serum lipids have a recognized association with atherogenesis, an important class of antiatherosclerotic agents are those with serum lipid-modifying effects. Serum lipids implicated in atherogenesis include serum cholesterol, serum triglycerides, and serum lipoproteins.

With respect to serum lipoproteins, at least three different classes of these substances have been characterized; high density lipoproteins (HDL's), low density lipoproteins (LDL's), and very low density lipoproteins (VLDL's). HDL's are often referred to as alphalipoproteins, while LDL's and VLDL's are referred to as betalipoproteins. The enhancement of HDL levels (hyperalphalipoproteinemic activity) is postulated to have direct antiatherosclerotic effects. See Eaton, R.P., J. Chron. Dis 31:131-135 (1978). In contrast, agents which reduce serum LDL's and serum VLDL's (hypobetalipoproteinemic agents) are also associated with antiatherogenic effects. See Haust, M.D., "Reaction Patterns of Intimal Mesenchyme to Injury and Repair in Atherosclerosis", Adv. Exp. Med. Biol. 43:35-57 (1974), which postulates that serum LDL is a factor in atherosclerotic lesion formation.

Numerous animal models have been developed for assessing antiatherosclerotic activity. Principal among these are models for assessing hypolipoproteinemic activity in the rat and antiatherosclerotic activity in the Japanese quail. For a description of the operation of the hypobetalipoproteinemic rat model, refer to the known methods of Schurr, P.E., et al., "High Volume Screening Procedure for Lypobetalipoproteinemia Activity in Rats", Adv. Exp. Med. Biol. 67: Atherosclerotic Drug Discovery, pp. 215-229, Plenum Press (1975). For a description of the Japanese quail model, see Day, C.E. et al., "Utility of a Selected Line (SEA) of the Japanese Quail (Corturnic Corturnix japonica) for the Discovery of New Anti-Atherosclerosis Drugs", Laboratory Animal Science 27:817-821 (1977).

2-Aminochromones (4H-1-benzopyran-4-ones) are known in the literature. For example, the antiallergic activity of 2-aminochromones has been described in the literature by Mazzei, Balbi, Ermili, Sottofattori and Roma (Mazzei, M., Ballbi, A., Ernili, A., Sottofattori, E., Roma, G., Farmaco. Ed. Sci., (1985) 40, 895 and Mazzei, M., Ermili, A., Balbi, A., Di Braccio, M., Farmaco. Ed. Sci., (1986) , 41, 611; CA 106:18313w). The CNS activity of 2-aminochromones has also been described (Balbi, A., Roma, G., Ermili, A., Farmaco. Ed. Sci., (1982) 37, 582; Ermili, A., Mazzei, M., Roma, G., Cacciatore, C., Farmaco. Ed. Sci., (1977), 32, 375 and 713). The nitro derivatives of various 2-aminochromones have recently been described (Balbi, A., Roma, G., Mazzei, M., Ermili, A., Farmaco. Ed. Sci., (1983) 38, 784) and Farmaco. Ed. Sci., 41(7), 548-57. 2-Amino-3-hydroxychromones are described in DE 2205913 and GB 1389186.

U.S. Patent 4,092,416 (see also DE 2555290 and CA 87:102383r) discloses various benzopyrone derivatives exhibiting anti-allergic activity, including 2-{2-[4-(2-methoxyphenyl)-piperazinyl-1]-ethyl}-5-methoxy-4-oxo-4H-1-benzopyran and 2-{2-[4-(2-methoxyphenyl)-piperazinyl-1]-ethyl}-5-(2-hydroxypropoxy)-4-oxo-4H-1-benzopyran.

JA-025657 and JP-259603 describe various 2-amino-3-carboxamide derivatives and 3-phenyl(optionally substituted)-2-aminochromone derivatives as useful as oncostatic and immunosuppressive agents.

The pharmacomodulation of α-adrenergic blocking agents by a series of benzopyrans, including 2-(1-piperidinylmethylene)-4H-1-benzopyran-4-one, is described in Eur. J. Med. Chem., 1987, 22(6), 539-44; CA 109:92718k.

Structurally, the closest compounds in the literature to 2-(4-morpholinyl)-4H-1-benzopyran-4-one (Cpd 2) is believed to be the 3-hydroxy, 3-methoxy and 3-acetyloxy analogues (i.e., 2-(4-morpholinyl)-3-hydroxychromone, 2-(4-morpholinyl)-3-methoxychromone and 3-(acetyloxy)-2-(4-morpholinyl)chromone) reported by Eiden and Docher (Eiden, F., Dolcher, D., Arch. Pharm. (Weinheim Ger.) (1975) 308, 385) and DE 2205913; CA 83(11):96942w and CA79(19):115440s. 6,7-dimethoxy-2-(4-morpholinyl)chromone is disclosed in J. Chem. Soc., Perkins Trans. 1, (2), 173-4; CA78(9) ;58275v. 3-Acetyl-2-(4-morpholinyl)chromone is disclosed in Arch. Pharm. 316(1), 34-42; CA98(15):12915g. 3-hydroxy-2-[4-(2-hydroxyethyl)-1-piperazinyl]-4H-1-benzopyran-4-one and 3-hydroxy-2-(4-methyl-1-piperazinyl)-4H-1-benzopyran-4-one are disclosed in Arch. Pharm 308(5), 385-8; CA83(11):96942w. 5,8-dimethoxy-2-(4-methyl-1-piperazinyl)-4H-1-benzopyran-4-one is disclosed in J. Heterocycl. Chem., 18(4), 679-84; CA95(17):150348v.

The synthesis of 2-aminochromones from the corresponding 2-sulphonyl and 2-sulphinyl analogues has been reported by Bantick and Suschitzky (Bantick, J.R., Suschitzky, J.L., J. Heterocyclic Chem., (1981) 18, 679). Also described in this report is the preparation of the HCL and H₂SO₄ salts of several 2-aminochromones.

The anti-platelet activity of some 2-(dialkylamino)chromones, namely: 2-(diethylamino)-5,6-dimethyl-4H-1-benzopyran-4-one,2-(diethylamino)-6,7-dimethyl-4H-1-benzopyran-4-one,2-(diethylamino)-7-hydroxy-5-methyl-4H-1-benzopyran-4-one, 2-(diethylamino)-5-hydroxy-7-methyl-4H-1-benzopyran-4-one, 2-(diethylamino)-6-chloro-8-isopropyl-4H-1-benzopyran-4-one, 2-(diethylamino)-5,7-methoxy-4H-1-benzopyran-4-one, 2-(ethylamino)-5-hydroxy-4H-1-benzopyran-4-one, 2-(ethylamino)-7-hydroxy-4H-1-benzopyran-4-one, 2-(diethylamino)-7-hydroxy-6-nitro-4H-1-benzopyran-4-one, 2-(diethylamino)-4H-1-benzopyran-4-one,2-(dimethylamino)-7-methoxy-4H-1-benzopyran-4-one,2-(diethylamino)-7-methoxy-4H-1-benzopyran-4-one, 2-(1-pyrro1idinyl)-7-methoxy-4H-1-benzopyran-4-one, 2-(1-piperidinyl)-7-methoxy-4H-1-benzopyran-4-one, 2-(diethylamino)-7-hydroxy-4H-1-benzopyran-4-one, 2-(1-piperdinyl)-7-hydroxy-4H-1-benzopyran-4-one,2-(ethylamino)-7-methoxy-4H-1-benzopyran-4-one, 2-(diethylamino)-5-hydroxy-4H-1-benzopyran-4-one, 2-(diethylamino)-5-methyl-8-isopropyl-4H-1-benzopyran-4-one, and 2-(diethylamino)-3-(4-morpohoinomethyl)-7-methoxy-4H-1-benzopyran-4-one, was reported by Mazzei et al. in Eur. J. Med. Chem. 23, 237-242 (May-June 1988); CA 110:75246h.

The literature on the use of an ynamine in the synthesis of a 2-aminochromones has been reported by Tronchet, Bachler and Bonenfant (Tronchet, J.M. J., Bachler, B., Bonenfant, A., Helv. Chim. Acta. (1976), 59, 941). In this report, a 2-amino-3-glycosylchromone was prepared.

2-Amino-1,3-benzoxazin-4-ones are also known in the literature. Specifically, 2-morpholinyl-4H-1,3-benzoxazin-4-one and 8-methyl-2-(4-morpholinyl)-4H-1,3-benzoxazin-4-one are described in Netherlands patent application 6,412,966 (see also U.S. 3,491,092), and in the literature (Grigat, E., Putter, R., Schneider, K., Wedemeyer, K., Chem. Ber., (1964) 97, 3036).

The fungicide and analgesic activity of 2-amino-1,3-benzoxazin-4-ones are also claimed by Sankyo in Japn. Tokkyo Koho 79 20,504 (CA 91:157755b) and in Japan (Kokai 72, 17,781 (CA 77:140107e). These patents appear to cover 2-(4-morpholinyl)-4H-1,3-benzoxazin-4-one and 6,7-substituted-2-(4-morpholinyl) analogues for the above indications.

The synthesis of 2-dialkylamino-1,3-benzoxazin-4-ones has been described by Kokel et al (see Tet. Letters (1984) 3837).

2-N-Alkyl and 2-N-aryl-1,3-benzoxazin-4-ones have been described by Palazzo and Giannola (Palazzo, S., Giannola, L.I., Atti. Accad. Sci. Lett. Arti Palermo, Parte 1, (1976) 34(2), 83-7).

2-Benzamidino-1,3-benzoxazin-4-one have been described by Brunetti, H., and Luthi, C.E. (in Helv. Chim. Acta., (1972) 55, 1566).

WO-A-9 006 921 (published 28 June 1990) discloses various 1-benzopyran-4-ones and 2-amino-1,3-benzoxazines-4-ones, including 2-(4-morpholinyl)-4H-1-benzopyran-4-one, 8-Methyl-2-(4-morpholinyl)-(7-phenylmethoxy)-4H-benzopyran-4-one, 7-[(1-cyclohexyl-1H-tetrazol-5-yl)methoxy]-8-methyl-2-(4-morpholinyl)-4H-1-benzopyran-4-one, 8-Methyl-2-(4-morpholinyl)-7-(2-(1-piperidinyl)ethyl)oxy-4H-1-benzopyran-4-one, 8-Methyl-2-(4-morpholinyl)-7-(2-(1-pyrrolidinyl)ethyl)oxy-4H-1-benzopyran-4-one, 7-[2-(ethylphenylamino)ethoxy]-8-methyl-2-(4-morpholinyl)-4H-1-Benzopyran-4-one, as well as their antiatherosclerotic, antithrombotic activity, cell proliferation (inhibitive) and/or inhibitive of platelet aggregation.

### BRIEF DESCRIPTION OF THE INVENTION

This invention relates to compounds of the Formula I which are useful in association with a pharmaceutical carrier as antiatherosclerotic agents. In addition, various compounds of the Formula I are useful inhibitors of cell proliferation and/or platelet aggregation.

### DETAILED DESCRIPTION OF THE INVENTION

The compounds of this invention are represented by Formula I (see Formula sheet) wherein
X is CZ where Z is H, C₁-C₅ alkyl, amino (-NH₂) or a halogen atom;
Y is -(CH₂)ₙNR₉R₁₀ wherein R₉ and R₁₀, being the same or different, are selected from the group consisting of
   (a) hydrogen, with the proviso that R₉ and R₁₀ are not both hydrogen;
   (b) C₁-C₁₂ alkyl;
   (c) phenyl optionally substituted with one, 2 or 3 C₁-C₄ alkyl, C₁-C₄ alkoxy, halo, OH, trifluoromethyl or -CO₂ (C₁-C₄ alkyl);
   (d) -(CH₂)ₙphenyl [wherein phenyl is optionally substituted with one, 2 or 3 C₁-C₄ alkyl, C₁-₄ alkoxy, halo, OH, trifluoromethyl or -CO₂(C₁-C₄alkyl)],
   (e) -(CH₂)ₙpyridinyl or
   (f) wherein R₉ and R₁₀, taken together with N, form a saturated or unsaturated heterocyclic amine ring selected from the group consisting of
      (aa) 4-morpholine optionally substituted with one or two members selected from the group consisting of C₁-C₄ alkyl, C₁-C₄ alkoxy, halo or trifluoromethyl,
      (bb) 4-thiomorpholine optionally substituted with one or two members selected from the group consisting of C₁-C₄ alkyl, C₁-C₄ alkoxy, halo or trifluoromethyl,
      (cc) 3-amino-1-pyrrolidine,
      (dd) 1-pyrrolidine optionally substituted with one or two members selected from the group consisting of C₁-C₄ alkyl, C₁-C₄ alkoxy, halo, OH, -CH₂OH, or trifluoromethyl,
      (ee) 1-piperidine optionally substituted with one or two members selected from the group consisting of C₁-C₄ alkyl, C₁-C₄ alkoxy, halo, trifluoromethyl, -(CH₂)_{q}OH, -CO₂H, -CO₂CH_{3,} -CO₂CH₂CH₃ or phenyl (wherein phenyl is optionally substituted with one, 2 or 3 C₁-C₄ alkyl, C₁-C₄ alkoxy, halo or trifluoromethyl),
      (ff) 1-piperazine, 4-(C₁-C₄alkyl)-1-piperazine (preferably 4-methyl-1-piperazine), 4-(cycloC₃-C₆alkyl)-1-piperazine, 4-phenyl-1-piperazine (wherein phenyl is optionally substituted with one, 2 or 3 C₁-C₄ alkyl, C₁-C₄ alkoxy, halo or trifluoromethyl) or 4-pyridinyl-1-piperazine optionally substituted with one or two members selected from the group consisting of C₁-C₄ alkyl, C₁-C₄ alkoxy, halo, OH, trifluoromethyl, -CH₂OH, -CO₂H, -CO₂C_{H} or -CO₂CH₂C_{H}
      (gg) thiazolidine, thiazolidine-4-carboxylic acid, pipecolinic acid, p-piperazinacetophenone, 1-homopiperazine, 1-methylhomopiperazine, 4-phenyl-1,2-3,6-tetrahydropyridine, proline, tetrahydrofurylamine, 1-(3-hydroxy)pyrrolidine, nipecotamide, 1,2,3,4-tetrahydroisoquinoline or imidazole;
and R₅, R₆, R₇ and R₈, being the same or different, are selected from the group consisting of hydrogen, C₁-C₈ alkyl, -(CH₂)ₙphenyl [wherein phenyl is optionally substituted with one, 2 or 3 C₁-C₄ alkyl, C₁-C₄ alkoxy, halo, OH, trifluoromethyl or -CO₂(C₁-C₄alkyl)], -(CH₂)ₙnaphthyl -(CH₂)ₙpyridinyl, -(CH₂)_{q}NR₉R₁₀, -CH=CH-phenyl [wherein phenyl is optionally substituted with one, 2 or 3 C₁-C₄ alkyl, C₁-C₄ alkoxy, halo, OH, trifluoromethyl or -CO₂(C₁-C₄alkyl)], -CH₂-CH=CH₂, -CH=CH-CH₃, -CH=CH₂, -O-CH₂-CH=CH₂, -C≡C-phenyl [wherein phenyl is optionally substituted with one, 2 or 3 C₁-C₄ alkyl, C₁-C₄ alkoxy, halo, OH, trifluoromethyl or -CO₂(C₁-C₄alkyl)], -O-(CH₂)ₚ(N-methylpiperidin-3-yl), -O-(CH₂)ₚNR₉R₁₀ [preferably -O-(CH₂)ₚ-4-(C₁-C₄alkyl)-1-piperazine, -O-(CH₂)ₚ(1-piperidinyl), -O-(CH₂)ₚ(1-pyrrolidinyl), more preferably -O-(CH₂)₂-4-methyl-1-piperazine], -O-CH₂CH(OCH₃)₂, -O-(CH₂)ₚOR₁₅ {wherein R₁₅ is selected from H, C₁-C₅ alkyl, -(CH₂)ₙphenyl [phenyl optionally substituted with one, 2 or 3 C₁-C₄ alkyl, C₁-C₄ alkoxy, halo, OH, trifluoromethyl or -CO₂(C₁-C₄alkyl)], -(CH₂)ₙpyridin-1-yl, -(CH₂)ₙpyridin-2-yl, -(CH₂)ₙpyridin-3-yl, -(CH₂)ₙpyridin-4-yl, -(CH₂)ₙ-1-(C₁-C₄alkyl)-1H-5-tetrazole, -(CH₂)ₙ-pyrimidine, -(CH₂)ₙ-2-benzoxazole, -(CH₂)ₙ-2-benzothiazole, -(CH₂)ₙ-(C₁-C₄alkyl)-triazole, -(CH₂)ₙ-(C₁-C₄alkyl)-imidazole}, -O-(CH₂)ₚ-O-(CH₂)ₚ-OR₁₅, -O-(CH₂)ₚ-S-R₁₅, -O-(CH₂)ₚ-O-(CH₂)ₚNR₉R₁₀, -O-(CH₂)ₚ-S-(CH₂)ₚNR₉R₁₀, -O-(CH₂)ₚ-S-(CH₂)ₚ-OR₁₅, -O-(CH₂)ₚ-S(O)-R₁₅, -O-(CH₂)ₚ-S(O₂)-R₁₅, -O(CH₂)ₚ-S(O)ₚ-S(O)-(CH₂)ₚNR₉NR₁₀, -O-(CH₂)ₚ-S(O)-(CH₂)ₚ-OR₁₅, -O-(CH₂)ₚ-S(O₂)-(CH₂)ₚNR₉R₁₀, -O-(CH₂)ₚ-S(O₂)-(CH₂)ₚ-OR₁₅, -O-(CH₂)ₚ-[4-[(CH₂)ₚOR₁₅]-1-piperazine], -O-(CH₂)ₚ-[4-(CH) (phenyl)₂-1-piperazine] [phenyl optionally substituted with one, 2 or 3 C₁-C₄ alkyl, C₁-C₄ alkoxy, halo, OH, trifluoromethyl or -CO₂(C₁-C₄alkyl)], -O-(CH₂)ₚ-[4-(CH₂)_{q}phenyl-1-piperazine] [phenyl optionally substituted with one, 2 or 3 C₁-C₄ alkyl, C₁-C₄ alkoxy, halo, OH, trifluoromethyl or -CO₂(C₁-C₄alkyl)], -O-(CH₂)ₚ-[4-(CH₂)_{q}pyridinyl-1-piperazine] [pyridinyl optionally substituted with one, 2 or 3 C₁-C₄ alkyl, C₁-C₄ alkoxy, halo, OH, trifluoromethyl, NR₉R₁₀ or -CO₂(C₁-C₄alkyl)], O-(CH₂)ₚ-[4-(NR₉R₁₀ substituted pyridinyl)-1-piperazine, -O-(CH₂)ₚ-(OH substituted 1-piperidine), -O-(CH₂)ₚ-1-pyrrolidin-2-one, -(CH₂)ₙC(O)-(CH₂)ₙR₉, -(CH₂)ₙC(O)O-(CH₂)ₚR₉, -(CH₂)ₙC(O)O-(CH₂)ₚNR₉R₁₀, -(CH₂)ₙC(O)(CH₂)ₙNR₉R₁₀, NO₂, -O-(CH₂)ₙC(O)-(CH₂)ₚR₉, -O-(CH₂)ₙC(O)O-(CH₂)ₚR₉, -O-(CH₂)ₙC(O)-(CH₂)ₙNR₉R₁₀, -NR₉R₁₀, -N(R₉)(CH₂)ₙC(O)-(CH₂)ₙR₁₀, -N(R₉)-(CH₂)ₙC(O)O-(CH₂)ₙR₁₀, N(R₉)(CH₂)ₙC(O)-(CH₂)ₙNR₉R₁₀, -O-(CH₂)ₙphenyl [wherein phenyl is optionally substituted with one, 2 or 3 C₁-C₄ alkyl, C₁-C₄ alkoxy, halo, OH, trifluoromethyl or -CO₂(C₁-C₄alkyl)], -O-(CH₂)ₙpyridine, -O(CH₂)ₙC(O)-(CH₂)ₙpyridine, -O-(CH₂)ₙC(O)O-(CH₂)ₙpyridine, -O-(CH₂)ₙC(O)-N(R₉)(CH₂)ₙpyridine, -O-(CH₂)ₙquinoxalinyl, -O-(CH₂)ₙquinolinyl, -O-(CH₂)ₙpyrazinyl, -O-(CH₂)ₙnaphthyl, -O-(CH₂)ₙC(O)-(CH₂)ₙnaphthyl, -O-(CH₂)ₙC(O)O-(CH₂)ₙnaphthyl, -O-(CH₂)ₙC(O)NR₉-(CH₂)ₙnaphthyl, halo (fluoro, chloro, bromo, iodo), OH, -(CH₂)_{q}-OH, (CH₂)_{q}OC(O)R₉, -(CH₂)_{q}OC-(O)-NR₉R₁₀, -(1-cyclohexyl-1H-tetrazol-5-yl)C₁-C₄ alkoxy, -[1-(C₁-C₅alkyl)-1H-tetrazol-5-yl]C₁-C₄ alkoxy (including -(1-cycloC₃-C₅ alkyl-1H-tetrazol-5-yl)C₁C₄alkoxy) , -[1-(phenyl)-1H-tetrazol-5-yl]C₁-C₄ alkoxy [wherein phenyl is optionally substituted with one, 2 or 3 C₁-C₄ alkyl, C₁-C₄ alkoxy, halo, OH, trifluoromethyl or -CO₂(C₁-C₄alkyl),-[1-(pyridinyl)-1H-tetrazol-5-yl]C₁-C₄ alkoxy, -[1-(1-phenylethyl)-1H-tetrazol-5-yl]C₁-C₄ alkoxy, -C₁-C₄ alkoxyl, or a group of Formula II (see Formula Sheet) wherein R' is methyl or carboxy, R" is hydrogen and R''' is selected from benzyl [optionally substituted with one, two or three groups selected from hydroxy, halogen or phenoxy (optionally substituted with one, two or three groups selected from hydroxy or halogen)], C₁-C₅ alkyl, -(CH₂)ₙCO₂H, -CH₂SH, -CH₂SCH₃, imidazolinylmethylene, indolinylmethylene, CH₃CH(OH), CH₂OH, H₂N(CH₂)₄-(optionally in protected form) or H₂NC(NH)NH(CH₂)₃ (optionally in protected form); with the overall proviso that at least one member of R₅, R₆, R₇ or R₈ is selected from the group consisting of -CH=CH₂, -O-(CH₂)ₚOH, -O-(CH₂)ₚ-O-(CH₂)ₙpyridin-2-yl,-O-(CH₂)ₚ-O-(CH₂)ₙpyridin-3-yl,-O-(CH₂)ₚ-O-(CH₂)ₙpyridin-4-yl, -O-(CH₂)ₚ-O-(CH₂)ₙ-1-(C₁-C₄alkyl)-1H-5-tetrazole, -O-(CH₂)ₚ-O-(CH₂)ₙ-pyrimidine, -O-(CH₂)ₚ-O-(CH₂)ₙ-2-benzoxazole, -O-(CH₂)ₚ-O-(CH₂)ₙ-2-benzothiazole, -O-(CH₂)ₚ-O-(CH₂)ₙ-(C₁-C₄alkyl)-triazole, -O-(CH₂)ₚ-O-(CH₂)ₙ-(C₁-C₄alkyl)-imidazole, -O-(CH₂)ₚ-O-(CH₂)ₚ-OR₁₅, -O-(CH₂)ₚ-S-R₁₅, -O-(CH₂)ₚ-O-(CH₂)ₚNR₉R₁₀, -O-(CH₂)ₚ-S-(CH₂)ₚNR₉R₁₀, -O-(CH₂)ₚ-S-(CH₂)ₚ-OR₁₅, -O-(CH₂)ₚ-S(O)-R₁₅, -O-(CH₂)ₚ-S(O₂)-R₁₅, -O-(CH₂)ₚ-S(O)-(CH₂)ₚNR₉R₁₀, -O-(CH₂)ₚ-S(O)-(CH₂)ₚ-OR₁₅, -O-(CH₂)ₚ-S(O₂)-(CH₂)ₚNR₉R₁₀, -O-(CH₂)ₚ-S(O₂)-(CH₂)ₚ-OR₁₅, -O-(CH₂)ₚ-[4-[(CH₂)ₚOR₁₅]-1-piperazine], -O-(CH₂)ₚ-[4-(CH) (phenyl)₂-1-piperazine] [phenyl optionally substituted with one, 2 or 3 C₁-C₄ alkyl, C₁-C₄ alkoxy, halo, OH, trifluoromethyl or -CO₂(C₁-C₄alkyl)], -O-(CH₂)ₚ-[4-(CH₂)_{q}phenyl-1-piperazine] [phenyl optionally substituted with one, 2 or 3 C₁-C₄ alkyl, C₁-C₄ alkoxy, halo, OH, trifluoromethyl or -CO₂(C₁-C₄alkyl)], -O-(CH₂)ₚ-[4-(CH₂)_{q}-pyridinyl-1-piperazine] [pyridinyl optionally substituted with one, 2 or 3 C₁-C₄ alkyl, C₁-C₄ alkoxy, halo, OH, trifluoromethyl, NR₉R₁₀ or -CO₂(C₁-C₄alkyl)], O-(CH₂)ₚ-[4-(NR₉R₁₀ substituted pyridinyl)-1-piperazine, -O-(CH₂)ₚ-(OH substituted 1-piperidine),-O-(CH₂)ₚ-1-pyrrolidin-2-one;
n is 0-5, preferably 0 or one;
p is 2-5, preferably 2 or 3;
q is 1-5, preferably 1 or 2;
and pharmaceutically acceptable salts thereof.

X is preferably CZ where Z is H or C₁-C₅ alkyl, more preferably H or methyl, most preferably H.

Y is preferably -(CH₂)ₙNR₉R₁₀ wherein R₉ and R₁₀ are taken together with N and form a saturated or unsaturated heterocyclic amine ring selected from definitions (aa), (bb), (cc), (dd), (ee) and (ff) above. More preferably, n is 0 or 1 (most preferably 0) and R₉ and R₁₀, taken together with N, form:
(aa) morpholine (preferably 4-morpholine) optionally substituted with one or two members selected from the group consisting of C₁-C₄ alkyl or phenyl (wherein phenyl is optionally substituted with one or 2 C₁-C₄ alkyl, C₁-C₄ alkoxy, halo or trifluoromethyl);
and preferably, at least one member selected from R₅, R₆, R₇ or R₈ is selected from the group consisting of -O-(CH₂)ₚ-S-R₁₅, -O-(CH₂)ₚ-[4-(CH)phenyl)₂-1-piperazine], -CH=CH₂, or -O-(CH₂)ₚO-(CH₂)ₚOR₁₅; more preferably,
(i) R₅, R₆, and R₇ are each hydrogen, and R₈ is selected from: -O-(CH₂)ₚ-[4-(CH)phenyl)₂-1-piperazine], or -O-(CH₂)ₚ-S-R₁₅; or
(ii) R₅ and R₆ are hydrogen, R₈ is hydrogen, halo, -CH=CH₂, or C₁-C₅ alkyl, and R₇ is selected from: -O-(CH₂)ₚ-[4-(CH)phenyl)₂-1-piperazine), or -O-(CH₂)ₚ-S-R₁₅.

X is most preferably CH.

Y is most preferably 4-morpholinyl.

R₈ is preferably hydrogen or C₁-C₅ alkyl, more preferably, hydrogen or methyl.

R₁₅ is preferably hydrogen, C₁-C₅ alkyl, -(CH₂)ₙphenyl, -(CH₂)ₙpyridin-2-yl or -(CH₂)ₙpyridin-3-yl.

Examples of preferred compounds include: Compounds 326 and 347; as well as salts thereof.

Accordingly the present invention includes the novel 2-amino(4H)-1-benzopyran-4-ones and 2-aminoalkyl(4H)-1-benzopyran-4-ones of Formula I and the antiatherosclerotic utility of said compounds.

The carbon content of various hydrocarbon containing moieties is indicated by a prefix designating the minimum and maximum number of carbon atoms in the moiety, i.e., the prefix Cᵢ-Cⱼ indicates a carbon atoms content of the integer "i" to the integer "j" carbon atoms, inclusive. Thus, C₁-C₃ alkyl refers to alkyl of 1-3 carbon atoms, inclucive, or methyl, ethyl, propyl, and isopropyl.

With respect to the above, C₁-C₄ alkyl is methyl, ethyl, propyl, or butyl, including isomeric forms thereof. Similarly, C₁-C₆ alkyl is methyl, ethyl, propyl, butyl, pentyl, hexyl, and isomeric forms thereof.

The term "halo" includes fluoro, chloro, bromo and iodo.

All temperatures throughout the specification are expressed in degrees Celcius (°C).

Examples of C₁-C₈ alkylthiomethyl are methylthiomethyl, ethylthiomethyl, propylthiomethyl, butylthiomethyl, pentylthiomethyl, hexylthiomethyl, and heptylthiomethyl, and isomeric forms thereof.

Examples of C₁-C₈ alkoxymethyl are methoxymethyl, ethoxymethyl, propoxymethyl, butoxymethyl, pentoxymethyl, butoxymethyl, pentoxymethyl, hexoxymethyl, and heptoxymethyl, and isomeric forms thereof.

Examples of heterocylic amines corresponding to heterocyclic amine rings according to -NR₉R₁₀ are:
4-morpholine,
4-phenyl-1-piperazine,
4-(2-pyridinyl)-1-piperazine,
2,6-dimethyl-4-morpholine,
1-pyrrolidine,
4-methyl-1-piperazine,
1-piperidine,
4-phenyl-1-piperidine
thiazolidine,
3-piperidine methanol,
2-piperidine methanol,
pipecolic acid,
3-piperidine ethanol,
2-piperidine ethanol,
1-piperazine propanol,
p-piperazinoacetophenone,
4-phenyl-1,2,3,6-tetrahydropyridine,
4-phenylpiperidine,
proline,
1-(3-hydroxy)pyrrolidine,
tetrahydrofurylamine,
pyrrolidimethanol,
3-pyrroline,
thiazolidine-4-carboxylic acid,
thiomorpholine,
nipecotamide,
2-methylpiperidine,
3-methylpiperidine,
4-methylpiperidine,
N-methylpiperazine,
1-methylhomopiperazine,
1-acetyipiperazine,
N-carboethoxypiperazine,
3-methylpiperazine-2-carboxylic acid,
2-methylpiperazine,
2,3,5,6,-tetramethylpiperazine,
1,4-dimethylpiperazine,
2,6-dimethylpiperazine,
2-methyl-1-phenylpiperazine,
1-(1-phenylethyl)piperazine,
1-(2-pyrazinyl)piperazine,
1-cyclopropylpiperazine,
1-cyclobutylpiperazine,
1,2,3,4-tetrahydroisoquinoline,
imidazole,
homopiperdine, and pharmaceutically acceptable salts and hydrates thereof.

Examples of -O(CH₂)ₚ(N-methylpiperdin-3-yl) include (2-(N-methylpiperdin-3-yl)ethyl)oxy, (3-(N-methylpiperdin-3-yl)propyl)oxy, (4-(N-methylpiperdin-3-yl)butyl)oxy.

Examples of -O-(CH₂)ₚNR₉R₁₀ include (2-(1-piperidinyl)ethyl)oxy, (2-(4-morpholinyl)ethyl)oxy, (2-(1-pyrrolidinyl)ethyl)oxy, (3-(N-methylpiperazinyl)propyl)oxy, (4-(N-ethyl-N-phenylamino)butyl)oxy,(5-(diethylamino)pentyl)oxy, (2-(4-benzylpiperazinyl)ethyl)oxy, and (3-(N,N-diisopropyl)propyl)oxy.

Examples of O-(CH₂)ₚOR₁₅ include (2-methoxyethyl)oxy, (3-butoxypropyl)oxy, (4-phenoxybutyl)oxy, (2-benzyloxyethyl)oxy, (2-(2-(1-piperidinyl)ethoxy)ethyl)oxy and (3-(3-picolylmethoxy)propyl)oxy.

Examples of -(CH₂)ₙpyridinyl include 2-pyridyl, 3-pyridylmethyl and 4-pyridylethyl.

Examples of -(CH₂)ₙpiperdinyl include 1-piperidinyl, 1-peiperidinylmethyl, 2-(1-piperidinyl) ethyl and 3-(1-piperidinyl)propyl.

Examples of -(CH2)qNR9R10 include (1-piperidinyl)methyl, 2-(4-morpholinyl)ethyl, 3-(1-pyrrolindinyl)propyl and 4-(1-piperazinyl) butyl.

Examples of -(CH₂)ₙC(O)-(CH₂)ₙR₉ include acetyl, acetylmethyl, methylacetylmethyl, methylacetylethyl, phenylacetyl, phenylacetylmethyl, 2-(phenylacetyl)ethyl, 2-pyridylacetyl, 3-pyridylacetylmethyl, 3-(t-butylacetyl)propyl and 4-(ethylacetyl)butyl.

Examples of -(CH₂)ₙC(O)O-(CH₂)ₚR₉ include carbomethoxy, carbomethoxymethyl, 2-(carbomethoxy)ethyl,carbophenylmethoxy, carbophenylmethoxymethyl, 2-(carbo(3-pyridyl)methoxy)ethyl, carboethoxymethyl and 3-(carbopropoxy)propoxy.

Examples of -(CH₂)ₙC(O)O-(CH₂)ₚNR₉R₁₀ include -C(O)O-(CH₂)₂N(ethyl)₂, -(CH₂₎C(O)O-(CH₂)₂N(CH₃)(phenyl), -(CH₂)₃C(O)O-(CH₂)₃(1-pyrrolidine), -(CH₂)₃C(O)O-(CH₂)₂(1-piperidinyl), and -(CH₂)C(O)O-(CH₂)₂(4-morpholinyl).

Examples of -(CH₂)ₙC(O)(CH₂)ₙNR₉R₁₀ include -(CH₂)C(O)(CH₂)N(ethyl)₂, -(CH₂)₂C(O)(CH₂)₂N(methyl)(phenyl), -C(O) (1-pyrrolidine) , -(CH₂)₂C(O)(CH₂)₃(1-piperidine), and -(CH₂)₃C(O)(CH₂)(4-morpholine).

Examples of -O-(CH₂)ₙC(O)-(CH₂)ₚR₉ include -O-(CH₂)C(O)-(CH₂)(CH₃), -O-C(O)-(CH₂)₂(CH₃), -O-(CH₂)₃C(O)-(CH₂)phenyl, -O-(CH₂)₂C(O)-(CH₂)₃(2-pyridyl), -O-(CH₂)C(O)-(CH₂)₂(3-pyridyl) and -O-(CH₂)₄C(O)-CH₂)₄(t-butyl).

Examples of -O-(CH₂)ₙC(O)O-(CH₂)ₚR₉ include -O-(CH₂)C(O)O-(CH₂)(CH₃), -O-C(O)O-(CH₂)₂(CH₃), -O-(CH₂)₂C(O)O-(CH₂)₃(phenyl) and -O-(CH₂)₃C(O)O-(CH₂)₂(3-pyridyl).

Examples of -O-(CH₂)ₙC(O)-(CH₂)ₙNR₉R₁₀ include -O-(CH₂)C-(O)-(CH₂)N(CH₃)₂, -O-C(O)-(CH₂)(1-pyrrolidine), -O(CH₂)C(O)-(1-piperidine), -O-(CH₂)₂C(O)-(CH₂)(1-N-methylpiperazine), -O-(CH₂)₂C(O)-(CH₂)₂(4-morpholine), -O-(CH₂)C(O)-(CH₂)₃(cyclohexylamine), -O-(CH₂)₂C(O)-(CH₂)₃(t-butylamine), -O-(CH₂)C(O)-(CH₂)₂(1-phenylethylamine), -O-(CH₂)C(O)-(CH₂)₂(aniline), -O-(CH₂)C(O)-(CH₂) (L-phenylalanine ethyl ester) and -O-(CH₂)₂nC(O)-(CH₂)₃(3-pyridylamine).

Examples of -N(R₉) (CH₂)C(O)-(CH₂)ₙR₁₀ include -N(CH₃)C(O)-(CH₃), -N(H) (CH₂)₂C(O)-(CH₂) (phenyl), -N(H)(CH₂)C(O)-(CH₂)₂(3-pyridyl) and -N(CH₃)(CH₂)₃C(O)-(CH₂)(CH₃).

Examples of -N(R₉)-(CH₂)ₙC(O)O-(CH₂)ₙR₁₀ include -N(H)-(CH₂)C(O)O-(CH₃), -N(H)-(CH₂)₂C(O)O-(CH₂)(benzyl), -N(H)-(CH₂)₂-C(O)O-(CH₂) (3-pyridyl) and -N(CH₃)-(CH₂)C(O)O-(CH₂)₂(t-butyl).

Examples of -N(R₉) (CH₂)ₙC(O)-(CH₂)ₙNR₉R₁₀ include -N(H)(CH₂)C(O)-(CH₂)N(CH₃)₂, -N(H)C(O)-(CH₂) (1-pyrrolidine), -N(H)(CH₂)₂C(O)-(CH₂)₂(1-piperidine), and -N(CH₃)(CH₂)C(O)-(CH₂)₂(4-morpholine).

Examples of -O-(CH₂)ₙphenyl include 2-(4-trifluoromethylphenyl)ethoxy, 4-chlorophenoxy, 4-fluorophenylmethoxy, 3-(4-methoxyphenyl)propoxy, 4-(2-methyl-4-fluorophenyl)butoxy, 2-(2-methoxyphenyl)ethoxy, 3-methoxyphenylmethoxy, 4-carbomethoxyphenylmethoxy, 2-(3,4-dichlorophenyl)ethoxy, 4-ethoxyphenylmethoxy, 3-(4-nitrophenyl)propoxy, 4-t-butylphenylmethoxy, 4-benzyloxyphenylmethoxy and 2-(3-triflouromethylphenyl)ethoxy.

Examples of -O-(CH₂)ₙpyridine include 2-pyridyloxy, 3-pyridylmethoxy and 2-(4-pyridyl)ethoxy.

Examples of -O(CH₂)ₙC(O)-(CH₂)ₙpyridine include -O(CH₂)C(O)-(CH₂)(2-pyridine), -O(CH₂)₃C(O) -(CH₂)(3-pyridine) and -O(CH₂)₂C(O)-(CH₂)₃(4-pyridine).

Examples of -O-(CH₂)ₙC(O)O-(CH₂)ₙpyridine include -O(CH₂)C(O)O-(CH₂)(2-pyridine),-O(CH₂)₃C(O)O-(CH₂)(3-pyridine) and -O(CH₂)₂C(O)O-(CH₂)₃(4-pyridine).

Examples of -O(CH₂)ₙC(O)-N(R₉)(CH₂)ₙpyridine include -O(CH₂)C(O)-N(CH₃)(CH₂)(2-pyridine), -O(CH₂)₂C(O)-N(CH₃)(CH₂)(3-pyridine) and -O(CH₂)C(O)-N(benzyl)(CH₂)₂(4-pyridine).

Examples of -O(CH₂)ₙquinoxilin include 2-quinoxalinyloxy, 2-quinoxalinylmethoxy and 2-(2-quinoxalinyl)ethoxy.

Examples of -O-(CH₂)ₙquinoliny include 2-quinolinyloxy, 2-quinolinylmethoxy and 2-(2-quinolinyl)ethoxy.

Examples of -O-(CH₂)ₙpyrazinyl include 2-pyrazinyloxy, 2-pyrazinylmethoxy and 2-(2-pyrazinyl)ethoxy.

Examples of -O-(CH₂)ₙnaphthyl include 1-naphthyloxy, 2-naphthylmethoxy and 2-(1-naphthyl)ethoxy.

Examples of -O-(CH₂)ₙC(O)-(CH₂)ₙnaphthyl include -O-(CH₂)C(O)-(CH₂)(1-naphthyl), -O-(CH₂)₂C(O)-(CH₂) (2-naphthyl), -O-C(O)-(CH₂)(1-naphthyl) and -O-(CH₂)2C(O)-(CH₂)₂(2-naphthyl).

Examples of -O-(CH₂)ₙC(O)O-(CH₂)ₙnaphthyl include -O-(CH₂)C(O)O-(CH₂) (1-naphthyl), -O(CH₂)₂C(O)O-(CH₂) (2-naphthyl), -O-C(O)O-(CH₂)(1-naphthyl) and -O-(CH₂)2C(O)O-(CH₂)₂(2-naphthyl).

Examples of -O-(CH₂)ₙC(O)NR₉-(CH₂)ₙnaphthyl include -O-(CH₂)C(O)N(H)(CH₂) (1-naphthyl), -O-(CH₂)C(O)N(CH₃)(CH₂)₂(2-naphthyl) and -O-(CH₂)C(O)N(benzyl)(CH₂)₃(1-naphthyl).

Examples of -(CH₂)_{q}-OH include hydroxymethyl, hydroxyethyl and hydroxybutyl.

Examples of (CH₂)_{q}OC(O)R₉ include (CH₂)OC(O)methyl, (CH₂)₂OC(O)ethyl, (CH₂)₃OC(O)phenyl, (CH₂)₄OC(O) (3-pyridyl) and (CH₂)OC(O)thiophene.

Examples of -(CH₂)_{q}OC(O)-NR₉R₁₀ include -(CH₂)OC(O)-N(CH₂)₂, -(CH₂)₂OC(O)-N(ethyl)₂, -(CH₂)₃OC(O)-(1-pyrrolidine), -(CH₂)₄OC(O)-(1-piperidine) and -(CH₂)OC(O)-N-benzyiamine.

Examples of -(1-cyciohexyl-1H-tetrazol-5-yl)C₁-C₄ alkoxy, -[1-(C₁-C₅alkyl)-1H-tetrazol-5-yl]C₁-C₄ alkoxy include -(1-cyclohexyl-1H-tetrazol-5-yl)methoxy, -(1-cyclohexyl-1H-tetrazol-5-yl)ethoxy, -[1-(methyl)-1H-tetrazol-5-yl]methoxy, -[1-(cyclopropyl)-1H-tetrazol-5-yl]ethoxy,-[1-(1-tert-butyl)-1H-tetrazol-5-yl]propoxy and -[1-(cyclopentyl)-1H-tetrazol-5-yl]methoxy.

Examples of -[1-(phenyl)-1H-tetrazol-5-yl]C₁-C₄ alkoxy (wherein phenyl is optionally substituted with one, 2 or 3 C₁-C₄ alkyl, C₁-C₄ alkoxy, halo or trifluoromethyl) include -[1-(phenyl)-1H-tetrazol-5-yl]methoxy,-[1-(phenyl)-1H-tetrazol-5-yl]ethoxy, -[1-(4-methoxyphenyl)-1H-tetrazol-5-yl]methoxy, -[1-(4-fluorophenyl)-1H-tetrazol-5-yl]propoxy.

Examples of -[1-(pyridinyl)-1H-tetrazol-5-yl]C₁-C₄ alkoxy or -[1-(1-phenylethyl)-1H-tetrazol-5-yl]C₁-C₄ alkoxy include -[1-(2-pyridinyl)-1H-tetrazol-5-yl]methoxy,-[1-(3-pyridinyl)-1H-tetrazol-5-yl]ethoxy, -[1-(4-pyridinyl)-1H-tetrazol-5-yl]propoxy, -[1-(1-phenylethyl)-1H-tetrazol-5-yl]methoxy, -[1-(1-phenylethyl)-1H-tetrazol-5-yl]ethoxy.

Examples of -(CH₂)ₙ-1-(C₁-C₄alkyl)-1H-5-tetrazole include -CH₂-1-methyl-1H-5-tetrazole, 1-methyl-1H-5-tetrazole, and -(CH₂)₂-1-methyl-1H-5-tetrazole.

Examples of -(CH₂)ₙ-pyimidine include -CH₂-pyrimidine, -(CH₂)₂-pyrimidine, pyrimidine.

Examples of-(CH₂)ₙ-2-benzoxazole include -(CH₂)-2-benzoxazole, -(CH₂)₂-2-benzoxazole and 2-benzoxazole.

Examples of -(CH₂)ₙ-2-benzothiazole include -(CH₂)-2-benzothiazole, -(CH₂)₂-2-benzothiazole, and 2-benzothiazole.

Examples of -(CH₂)ₙ-(C₁-C₄alkyl)-triazole include -(CH₂)-methyl-triazole, -(CH₂)₂-methyl-triazole, and -methyl-triazole.

Examples of -(CH₂)ₙ-(C₁-C₄alkyl)-imidazole include -(CH₂)-methyl-imidazole, -(CH₂)₂-methyl-imidazole, and -methyl-imidazole.

Examples of -O-(CH₂)ₚ-O-(CH₂)ₚ-OR₁₅ include -O-(CH₂)₂-O-(CH₂)₂-O-benzyl, -O-(CH₂)₂-O-(CH₂)₂-O-methyl, -O-(CH₂)₂-O-(CH₂)₂-O-phenyl, and -O-(CH₂)₂-O-(CH₂)₂-O-pyridinyl.

Examples of -O-(CH₂)ₚ-S-R₁₅ include -O-(CH₂)₂-S-1-methyl-1H-5-tetatrazole, -O-(CH₂)₂-S-pyrimidine, -O-(CH₂)₂-S-pyridine, -O-(CH₂)₂-S-benzyl.

Examples of -O-(CH₂)ₚ-O-(CH₂)ₚNR₉R₁₀ include -O-(CH₂)₂-O-(CH₂)₂-1-piperidine, -O-(CH₂)₂-O-(CH₂)₂-4-methyl-1-piperazine, -O-(CH₂)₂-O-(CH₂)₂-diethylamine, and -O-(CH₂)₂-O-(CH₂)₂-4-pyridinyl-1-piperazine.

Examples of -O-(CH₂)ₚ-S-(CH₂)ₚNR₉R₁₀ include -O-(CH₂)₂-S-(CH₂)₂-1-piperidine, -O-(CH₂)₂-S-(CH₂)₂-4-methyl-1-piperazine, -O-(CH₂)₂-S-(CH₂)₂-diethylamine, -O-(CH₂)₂-S-(CH₂)₂-4-pyridinyl-1-piperazine.

Examples of -O-(CH₂)ₚ-S-(CH₂)ₚ-OR₁₅ include -O-(CH₂)₂-S-(CH₂)₂-O-benzyl, -O-(CH₂)₂-S-(CH₂)₂-O-methyl, -O-(CH₂)₂-S-(CH₂)₂-O-phenyl, -O-(CH₂)₂-S-(CH₂)₂-O-pyridinyl.

Examples of -O-(CH₂)ₚ-S(O)-R₁₅ include -O-(CH₂)₂-S(O)-1-methyl-1H-5-tetatrazole, -O-(CH₂)₂-S(O)-pyrimidine, -O-(CH₂)₂-S(O)-pyridine, and -O-(CH₂)₂-S(O)-benzyl.

Examples of -O-(CH₂)ₚ-S(O₂)-R₁₅ include -O-(CH₂)₂-S(O₂)-1-methyl-1H-5-tetatrazole, -O-(CH₂)₂-S(O₂)-pyrimidine, -O-(CH₂)₂-S(O₂)-pyridine, and -O-(CH₂)₂-S(O₂)-benzyl.

Examples of -O-(CH₂)ₚ-S(O)-(CH₂)ₚNR₉R₁₀ include -O-(CH₂)₂-S(O)-(CH₂)₂-1-piperidine, -O-(CH₂)₂-S(O)-(CH₂)₂-4-methyl-1-piperazine, -O-(CH₂)₂-S(O)-(CH₂)₂-diethylamine, and -O-(CH₂)₂-S(O)-(CH₂)₂-4-pyridinyl-1-piperazine.

Examples of -O-(CH₂)ₚ-S(O)-(CH₂)ₚ-OR₁₅ include -O-(CH₂)₂-S(O)-(CH₂)₂-O-benzyl,-O-(CH₂)₂-S(O)-(CH₂)₂-O-methyl, -O-(CH₂)₂-S(O)-(CH₂)₂-O-phenyl, and -O-(CH₂)₂-S(O)-(CH₂)₂-O-pyridinyl.

Examples of -O-(CH₂)ₚ-S(O₂)-(CH₂)ₚNR₉R₁₀ include -O-(CH₂)₂-S(O₂)-(CH₂)₂-1-piperidine, -O-(CH₂)₂-S(O₂)-(CH₂)₂-4-methyl-1-piperazine, -O-(CH₂)₂-S(O₂)-(CH₂)₂-diethylamine, and -O-(CH₂)₂-S(O₂)-(CH₂)₂-4-pyridinyl-1-piperazine.

Examples of -O-(CH₂)ₚ-S(O₂)-(CH₂)ₚ-OR₁₅ include -O-(CH₂)₂-S(O₂)-(CH₂)₂-O-benzyl, -O-(CH₂)₂-S(O₂)-(CH₂)₂-O-methyl, -O-(CH₂)₂-S(O₂)-(CH₂)₂-O-phenyl, and -O-(CH₂)₂-S(O₂)-(CH₂)₂-O-pyridinyl.

Examples of -O-(CH₂)ₚ-[4-[(CH₂)ₚOR₁₅]-1-piperazine] include -O-(CH₂)₂-[4-[(CH₂)₂OH]-1-piperazine, -O-(CH₂)₂-[4-[(CH₂)₂Obenzyl]-1-piperazine], and -O-(CH₂)₂-[4-[(CH₂)₂Opyridinylmethyl]-1-piperazine].

Examples of -O-(CH₂)ₚ-[4-(CH)(phenyl)₂-1-piperazine] include -O-(CH₂)₂-[4-(CH)(phenyl)(p-chlorophenyl)-1-piperazine], -O-(CH₂)₂-[4-(CH)(phenyl)₂-1-piperazine], and -O-(CH₂)₂-[4-(CH)(p-fluorophenyl)₂-1-piperazine].

Examples of -O-(CH₂)ₚ-[4-(CH₂)_{q}phenyl-1-piperazine] include -O-(CH₂)₂-[4-(CH₂)phenyl-1-piperazine], -O-(CH₂)₂-[4-(CH₂)₂phenyl-1-piperazine], and -O-(CH₂)₂-[4-(CH₂)-m-trifluoromethylphenyl-1-piperazine].

Examples of -O-(CH₂)ₚ-[4-(CH₂)_{q}pyridinyl-1-piperazine] include -O-(CH₂)₂-[4-(CH₂)pyridinyl-1-piperazine], and -O-(CH₂)₂-[4-(CH₂)₂pyridinyl-1-piperazine].

Examples of -O-(CH₂)ₚ-[4-(NR₉R₁₀ substituted pyridinyl)-1-piperazine] include -O-(CH₂)₂[4-(3-ethylamino-2-pyridinyl)-1-piperazine, -O-(CH₂)₂-[4-(3-piperidinyl-2-pyridinyl)-1-piperazine, and -O-(CH₂)₂-[4-(3-amino-2-pyridinyl)-1-piperazine.

Examples of -O-(CH₂)ₚ-(OH substituted 1-piperidine) include -O-(CH₂)₂-(4-hydroxy-1-piperidine) and -O-(CH₂)₂-(3-hydroxy-1-piperidine).

Examples of -O-(CH₂)ₚ-1-pyrrolidin-2-one include -O-(CH₂)₂-1-pyrrolidin-2-one and -O-(CH₂)₃-1-pyrrolidin-2-one.

Examples of optionally substituted piperazines include 2-hydroxymethyl4-methyl-1-piperazine, 2-carboxy-4-phenyl-1-piperazine, 2-methoxy-1-piperazine, 3-methyl-4-phenyl-1-piperazine, and 2-carbomethoxy-4-methyl-1-piperazine.

The subject specification and claims include the N-oxides of the tertiary amines and aromatic heterocyclic amines defined herein.

Pharmaceutically acceptable salts means salts useful for administering the compounds of this invention and include mesylate, hydrochloride, hydrobromide, hydroiodide, sulfate phosphate, acetate, propionate, lactate, maleate malate succinate, tartrate, and the like. These salts may be in hydrated form.

The compounds of Formula I are all characterized by pronounced antiatherogenic activity, rendering these compounds useful in the treatment and prophylaxis of atherosclerosis.

Preferred antiatherosclerotic compounds include Compound 326.

In addition, various compounds of Formula I are also potent inhibitors of cell proliferation and are contemplated as useful in the treatment of proliferative diseases such as cancer, rheumatoid arthritis, psoriasis, pulmonary fibrosis, scleroderma, cirrhosis of the liver and for the improved utilization of artificial prosthetic devices such as arterial grafts. These agents may also be useful in the prevention or treatment of obstruction or restenosis of arteries by subsequent administration of drug in cases such as by-pass surgery, coronary by-pass surgery, balloon angioplasty (and other procedures directed at re-establishing patency in occluded or partly occluded vessels, i.e atherectomy, laser or ultrasonic procedures), transplants, and post-thrombotic re-stenosis.

Compounds of Formula I which are inhibitors of cell proliferation are those active in the test procedure described in Pledger W.J., Stiles C.D., Antniades H.N., Scher C.D. [Proc. Natl. Acad. Sci (USA) (1977). Examples of inhibitors of cell proliferation include Compounds 217, 219, 222, 223, 226, 229, 230, 234-238, 242, 244, 246, 253, 269, 296 and 326.

In addition, various compounds of Formula I are also inhibitors of ADP-induced platelet aggregation and are useful in the prevention or treatment of thrombotic diseases and related complications by, for example, inhibition or reversal of platelet aggregation, or platelet adhesion or blood coagulation.

Compounds which are inhibitors of platelet aggregation are those active in the test procedure described in Born, G.R., Cross M.J., J. Physiol., 168, p. 178 (1963). Examples of inhibitors of ADP-induced platelet aggregation include: Compounds 217, 219, 223, 246, 253, 269, 296, 326-331, 333, 335 and 347.

Several of these compounds, including compound 347, have been found to significantly inhibit platelet thrombus formation in a canine model of platelet-dependent coronary thrombus formation. Shebuski, R.J., Ramjit, D.R., Bencen, G.H. and Polokoff, M.A. J. Biol. Chem. 264:21550, 1989. Compound 239 accelerates the rate of thrombolysis and prevents reocclusion following successful thrombolysis in a canine model of coronary thrombosis. Shebuski, R.J., Stabilito I.J., Sitko, G.R., and Polokoff, M.H., Circulation 82:169-177, 1990.

In addition, various compounds of Formula I are also potent vasodilators and are useful in the treatment of hypertension, peripheral vascular disease, vascular complications of diabetes and tissue ischemia due to poor blood flow or poor oxygen delivery. Compounds of Formula I which are vasodilators are those active in the test procedure described in Papadopoulos S.M., Gilbert B.A., Webb R.C., D'Amato C.J. [Neurosurgery 26:2605-2608 (1990)] using phenylephrine and other constricting agents in addition to endothelin. Examples of inhibitors of vasoconstrictors include compounds 223, 227, 231, 234, 237, 269 and 347.

Accordingly, in using compounds of Formula I for the prevention or treatment of atherosclerotic disease or thrombotic diseases, an oral route of administration, either by conventional oral dosage forms or by mixture with food, represents the preferred method of their systemic administration. Alternatively, however, these compounds may be administered by other convenient routes of administration whereby systemic activity is obtained. These other routes of administration would include rectal, vaginal, subcutaneous, intramuscular, intravenous, and like routes.

In using compounds of Formula I for use in angioplasty, an oral route of administration represents the preferred method of their systemic administration. Alternatively, however, these compounds may be administered by other convenient routes of administration whereby systemic activity is obtained.

The patient or animal being treated must be given periodic doses of the drug in amounts effective to reduce serum and/or arterial cholesterol, and reduce arterial atherosclerotic lesion size (as determined by angiogram, ultrasound, NMR, etc.); or, by the inhibition or reversal of platelet aggregation, platelet adhesion or blood coagulation; or, by preventing arterial occlusion in vascular trauma associated with procedures such as by-pass grafts, coronary by-passes, angioplasty, post-thrombotic re-stenosis and transplants.

Such effective dosages are readily determined by methods known in the art. For example, small daily doses of the drug ( e.g., 0.01-200 mg/kg) may be administered initially with higher succeeding doses until levels of serum and/or arterial cholesterol are favorably affected. By this regimen, a compound of Formula I is administered initially at doses as low as about 0.01 mg/kg per patient per day, with increasing doses up to about 200 mg/kg per patient per day. In the event the antiatherogenic response in a patient being treated at a dose of 200 mg/kg per day is insufficient, higher doses are also utilized to the extent patient tolerance permits further increases in dose.

While the preferred dosage regimen is with single daily dosing of patients, also preferred for obtaining more uniform serum levels of drug are multiple dosages per day (e.g., up to 4-6 times daily). Accordingly, when 4 daily doses of drug are to be administered, each such dose may be about 50 mg/kg per patient per dose, or higher depending on tolerance.

Similar doses are employed in hon-human mammals, e.g. 0.01-200 mg/kg/day.

Charts A, E, G, I, J, K and L herein describe various methods by which the compounds of Formula I are prepared. With respect to these Charts, X, Y, R₅, R₆, R₇, R₈, R₉ and R₁₀ are as defined above.

With respect to Chart A, the compounds of Formula I are prepared by mixing the salicylic acid ester with the morpholine ynamine neat, or in an organic solvent, with stirring. After several minutes, a tertiary amine base, e.g. TEA (triethylamine), is added and the reaction stirred for a period of time. The product can be isolated by recrystallization or column chromatography.

With respect to Chart E, these compounds can be prepared by treatment of a o-hydroxy acetophenone with an iminium salt such as morpholine-4-phosgene iminium chloride, in the presence of boron trifluoride etherate. Subsequent hydrolysis and alkylation yields the desired compounds.

With respect to Chart G, the treatment of an o-hydroxy acetophenone containing a halogen group with an iminium salt such as 4-morpholine dichloromethyleniminium chloride, in the presence of boron trifluoride etherate. Subsequent hydrolysis and alkylation yields the 2-aminochromone. Treatment of the 2-aminochromone with a tetraalkyl tin reagent in the presence of a palladium catalyst such as (bis)triphenylphosphine palladium dichloride and a salt such as lithium chloride affords a 2-aminochromone substituted with an alkyl substituent.

With respect to Chart I, the compounds of formula I are prepared by treating 4-benzyloxy-2-hydroxy-3-methylacetophenone with sodium hydride, then ethyl α-methylthioacetate and finally acid to yield 7-benzyloxy-8-methyl-2-methylthiomethyl-4H-[1]-benzopyran-4-one. Treatment of that compound with methyl iodide affords the corresponding 7-benzyloxy-8-methyl-2-iodomethyl-4H-[1]-benzopyran-4-one. Treatment of that compound with the appropiate amine then afforded the compounds of formula I. Compounds of formula I were also prepared by treating a formula I compound such as 7-benzyloxy-8-methyl-2-(4-morpholiniylmethyl) -4H-[1]-benzopyran-4-one with a transition metal catalyst in an atmosphere of hydrogen to yield 7-hydroxy-8-methyl-2-(4-morpholiniylmethyl)-4H-[1]-benzopyran-4-one. Alkylation of that phenol with the appropiate group also afforded compounds of formula I.

Alternatively, compounds of formula I can also be prepared by hydrogenation of a R₅₋₈ benzyloxy 2-amino-4H-1-benzopyran-4-one followed by alkylation of the resulting phenol as illustrated in chart H.

With respect to Chart J, these compounds are prepared by initial alkylation of the appropriate 2-aminochromone phenol (e.g. prepared according to the methods of Charts D or E) with 1,2-dibromoethane under phase transfer catalysis. Direct substitution of the bromine with an appropriate amine nucleophile affords the 2-aminochromone with a 2-aminoethyloxy substitutent.

With respect to Chart K, these compounds are prepared by treatment of a O-hydroxyacetophenone with potassium t-butoxide and an α-amino acetate, such as methy-2-(4-morpholinyl)-acetate, followed by acidification of the initial adduct.

With respect to Chart L, a procedure for the preparation of 2-aminochromones is contemplated in which a salicylic ester is treated with the anion of an acetyl amine (such as from lithium diisopropyl amide deprotonation of acetyl-4-morpholine) to afford an initial β-ketoamide. This compound, upon cyclodehydration with a reagent such as polyphosphoric ester, would give the 2-aminochromone.

The synthesis of the compounds of the present invention is more completely understood by the following Examples.

Following the general procedure of Example 39 of WO-A-9006921, but employing the appropriate hydroxyacetophenone instead of 2',4'-dihydroxy-3'-methylacetophenone, the following products are prepared:
- Cpd 219: 8-methyl-7-[(2-thiomethyl)ethyl]oxy-2-(4-morpholinyl) -4H-1-benzopyran-4-one, mp. 182.5-184;
- Cpd 221: 8-methyl-7-[(2-phenylmethoxy)ethyl]oxy-2-(4-morpholinyl)-4H-1-benzopyran-4-one, mp. 155-156;
- Cpd 222: 7-[2-(hydroxy)ethyl]oxy-8-methyl-2-(4-morpholinyl)-4H-1-benzopyran-4-one, mp. 267-268;
- Cpd 333: 7-[2-(2-methoxyethoxy)ethoxy]-8-methyl-2-(4-morpholinyl)-4H-1-benzopyran-4-one, mp. 127-128.

Following the general procedure of Example 194 of WO-A-9006921, but starting with the appropriate 2'-hydroxyacetophenone instead of 2',4'-dihydroxy-3'-iodoacetophenone, there are prepared the following products:
- Cpd 217: 2-(4-morpholinyl)-7-phenylmethoxy-8-vinyl-4H-1-benzopyran-4-one, mp. 181-182.5;
- Cpd 326: 8-ethenyl-7-[2-(4-methyl-l-piperazinyl)-ethoxy]-2-(4-morpholinyl)-4H-1-Benzopyran-4 -one, mp. 151-152;
- Cpd 327: 8-ethenyl-2-(4-morpholinyl)-7-[2-(1-piperidinyl)ethoxy]-4H-1-benzopyran-4-one, mp. 161-162;
- Cpd 328: 8-ethenyl-1-(4-morpholinyl)-7-[2-(4-phenyl-1-piperidinyl)ethoxy]-4H-1-benzopyran-4-one, mp. 169-169.5 ;
- Cpd 329: 8-ethenyl-2-(4-morpholinyl)-7-[2-(1-pyrrolidinyl)ethoxy]-4H-1-benzopyrany-4-one, mp. 155-156;
- Cpd 330: 8-ethenyl-2-(4-morpholinyl)-7-[2-(4-thiomorpholinyl)ethoxy]-4H-1-benzopyran-4-one, mp. 210.5-211.5; and
- Cpd 331: (R)-8-ethenyl-7-[2-[2-(hydroxymethyl)-1-pyrrolidinyl]ethoxy]-2-(4-morpholinyl)-4H-1-benzopyran-4-one, mp. 115-117.

### Preparation 1 Preparation of 7-(2-Bromoethyl)oxy-8-methyl-2-(4-morpholinyl)-4H-1-benzopyran-4-one (Relating to Chart J)

### Part A

7-Hydroxy-2-(4-morpholinyl)-8-methyl-4H-1-benzopyran-4-one (13,1g) is suspended in 150ml of 50% sodium hydroxide in a 500ml flask. The mixture is treated successively with 2.8g (8.2 mmol) tetrabutylmmonium hydrogen sulfate and 50ml (0.58 mol) of 1,2-dibromoethane. The reaction mixture is warmed to 60°C for 2h and cooled to 0°C. The solid is collected and washed well with 2N NaoH, water and ether. The material is dissolved in chloroform, adsorbed onto 30g of silica gel (230-400 mesh) and chromatographed over 400g silica gel, eluting with 4% methanol/methylene chloride to afford 8.1g (40%) of 7-(2-Bromoethyl)oxy-8-methyl-2-(4-morpholinyl)-4H-1-benzopyran-4-one, mp. 210-211.5°C

### Part B

### Preparation of 7-[2-(4-Methyl-1-piperazinyl)ethyl]oxy-8-methyl-2-(4-morpholinyl)-4H-1-benzopyran-4-one

7-(2-Bromoethyl)oxy-8-methyl-2-(4-morpholinyl)-4H-1-benzopyran-4-one (4.0g, 10.9 mmol) is suspended in 10ml chloroform and 7ml of N-methyl piperazine is added. The reaction is warmed to reflux for 5h and cooled to room temperature. The mixture is partitioned between 50ml of 1:1 2N NaOH/saturated NaCl and 25ml of methylene chloride. The combined organics were dried over magnesium sulfate, concentrated in vacuo, and chromatographed over 80g silica gel, eluting with 15% methanol/dichlormethane to afford 3.5g (83%) of cpd 208, mp. 159-159.5, after recrystallization from ethyl acetate.

Following the general procedure of Preparation 1 (Part B), using the appropriate bromoethyloxy-2-(4-morpholinyl)-4H-1-benzopyran-4-one and employing the appropriate amine, alcohol or sulfide nucleophile, there are prepared the following products:
- Cpd 220: 8-Methyl-2-(4-morpholinyl)-7-[2-(4-(2-hydroxy)ethyl-1-piperazinyl)ethyl]oxy-4H-1-benzopyran-4-one, mp. 192.5-193.5;
- Cpd 223: 8-Methyl-2-(4-morpholinyl)7-[2-(2-thiopyridinyl)ethyl]oxy-4H-1-benzopyran-4-one, mp. 146-147;
- Cpd 226: 8-Methyl-7-[2-((1-Methyl-1,3-imidazol-2-yl))thio)ethyl]oxy-2-(4-morpholinyl) -4H-1-benzopyran-4-one, mp. 170-170.5;
- Cpd 227: 7-[2-((Bis-N,N'-(2-methoxy)ethoxy)amino)ethyl]oxy-8-methyl-2-(4-morpholinyl)-4H-1-benzopyran-4-one, mp. 88-89 ;
- Cpd 228: 8-Methyl-7-[2-((4-Methyl-1,2,4-triazol-3-yl)thio)ethyl]oxy-2-(4-morpholinyl)-4H-1-benzopyran-4-one, mp. 221-221.5;
- Cpd 229: 7-[2-(N-Ethyl-N'-((2-hydroxy)ethyl)amino)ethyl]oxy-8-methyl-2-(4-morpholinyl)-4H-1-benzopyran-4-one,mp. 144-145;
- Cpd 230: 8-Methyl-7-[2-((1-Methyl-5-tetrazoyl)thio)ethyl]oxy-2-(4-morpholinyl)-4H-1-benzopyran-4 -one, mp. 188.5-189.5;
- Cpd 231: 8-Methyl-2-(4-morpholinyl)-7-[2-((2-pyrimidinyl)thio)ethyl]oxy-4H-1-benzopyran-4-one, mp. 202.5-203.5;
- Cpd 234: 7-[2-((2-(Bis-N,N'-diethylamino)ethyl)thio)ethyl]oxy-8-Methyl-2-(4-morpholinyl)-4H-1-benzopyran-4-one, mp. 101-103;
- Cpd 235: 8-Methyl-7-[2-((2-benzoxazolyl)thio)ethyl]oxy-2-(4-morpholinyl)-4H-1-benzopyran-4-one, mp. 221-222;
- Cpd 236: 8-Methyl-7-[2-((2-benzothiazolyl)thio)ethyl]oxy-2-(4-morpholinyl)-4H-1-benzopyran-4-one, mp. 185.5-187;
- Cpd 237: 7-[2-(4-(3-Ethylamino-pyridin-2-yl)-1-piperazinyl)ethyl]oxy-8-methyl-2-(4-morpholinyl)-4H-1-benzopyran-4-one, mp. 168-169;
- Cpd 238: 8-Methyl-2-(4-morpholinyl)-7-[2-(pyrrolidinone-1-yl)ethyl]oxy-4H-1-benzopyran-4-one, mp. 144.5-145.5; and
- Cpd 335: 8-methyl-2-(4-morpholinyl)-7-[2-(phenylthio)ethoxy]-4H-1-benzopyran-4-one, mp. 158-159;
- Cpd 242: 2-(4-morpholinyl)-8-[2-(2-pyridinylthio)ethoxy]-4H-1-benzopyran-4 -one, mp. 148-149;
- Cpd 244: 8-[2-[4-[3-(ethylamino)-2-pyridinyl]-1-piperazinyl]ethoxy]-2-(4-morpholinyl)-4H-1-benzopyran-4-one, mp. 122-123;
- Cpd 246: 8-methyl-2-(4-morpholinyl)-7-[2-(phenylsulfinyl)ethoxy] -4H-1-benzopyran-4-one, mp. 202-203;
- Cpd 253: 7-[2-[(2-methoxyphenyl)thio]ethoxy-8-methyl-2-(4-morpholinyl)-4H-1-benzopyran-4-one, mp. 155-156;
- Cpd 269: 8-methyl-2-(4-morpholinyl)-7-[2-(2-pyridinyloxy)ethoxy]-4H-1-benzopyran-4-one, mp. 175-175.5;
- Cpd 296: 2-(4-morpholinyl)-8-[2-(4-pyridinyloxy)-1-piperazinyl]ethoxy]-4H-1-benzopyran-4-one, mp. 151.5-152.5;
- Cpd 297: 8-[2-[4-[(4-chlorophenyl)phenylmethyl]-1-piperazinyl]ethoxy]-2-(4-morpholinyl)-4H-1-benzopyran-4-one, mp. 167-168; and
- Cpd 347: 7-[2-(4-ethyl-1-piperazinyl)ethyl]oxy-8-methyl-2-(4-morpholinyl)-4H-1-benzopyran-4-one, mp. 144.5-145.5.

### Preparation 2 Preparation of 7-[2-(4-methyl-1-piperazinyl)ethyl]oxy-8-methyl-2-(4-morpholinyl)-4H-1-benzopyran-4-one, mesylate salt

7-[2-(4-Methyl-1-piperazinyl)ethyl]oxy-8-methyl-2-(4-morpholinyl)-4H-1-benzopyran-4-one (Preparation 1; 2.0 g, 5.16 mmol) is dissolved in 25 ml of methylene chloride under nitrogen. The solution is diluted with 5 ml of methanol and treated with 0.335 ml (5.16 mmol) of methanesulfonic acid. The mixture is concentrated in vacuo to a residual foam. The foam is crystallised from 25 ml of ethyl acetate and allowed to digest overnight. The off-white solid is collected, washed with ether and dried in vacuo for 6 h at room temperature and for 24 h at 50°C to afford 2.48 g (99%) of the title salt (mp. 207.5-208.5).

Following the general procedure of Preparation 2, the following salt was prepared:
- Cpd 344: 8-methyl-2-(4-morpholinyl)-7-[2-(3-pyridinyloxy)ethoxy]-4H-1-benzopyran-4-one, bismesylate salt, mp. 175-177.

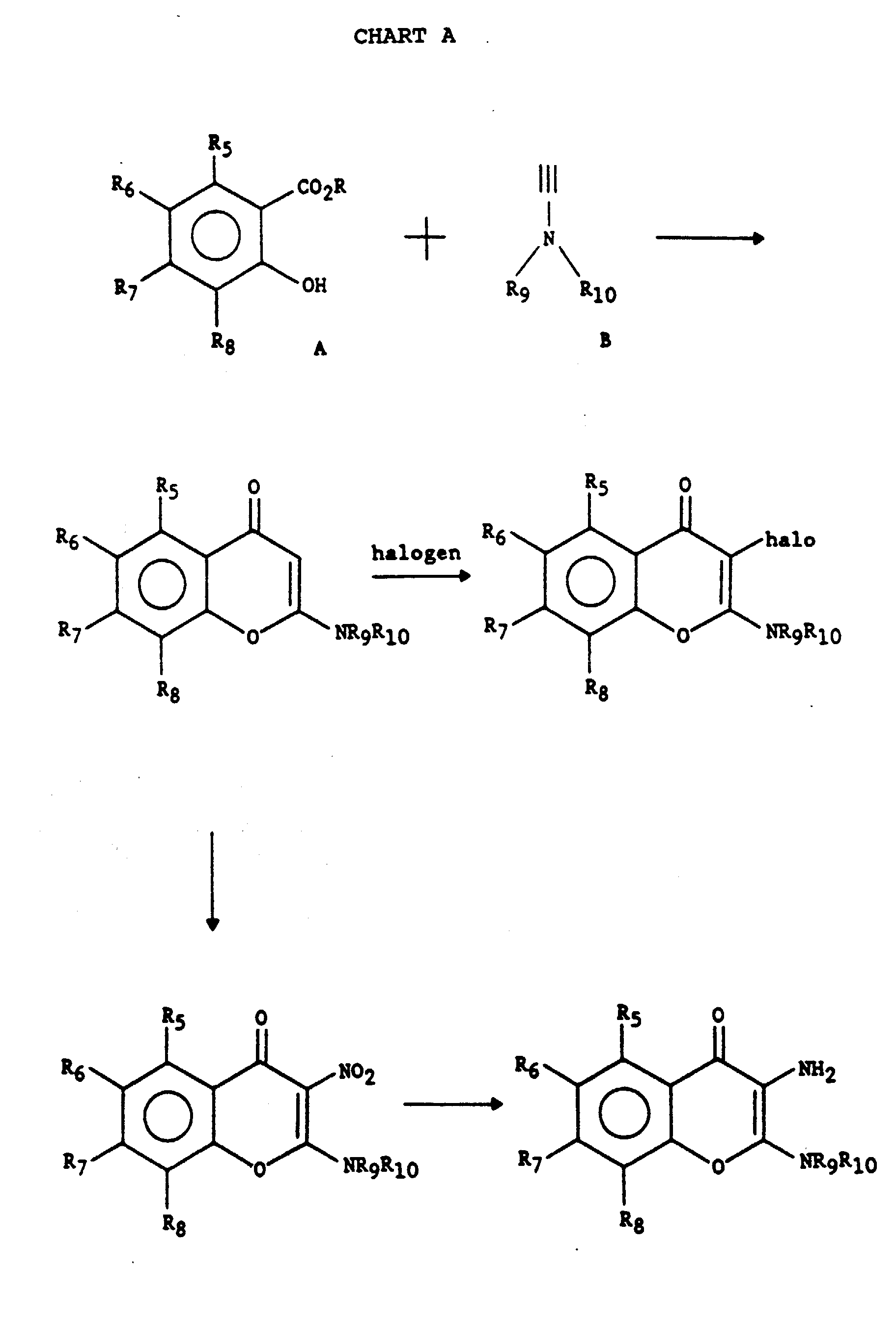

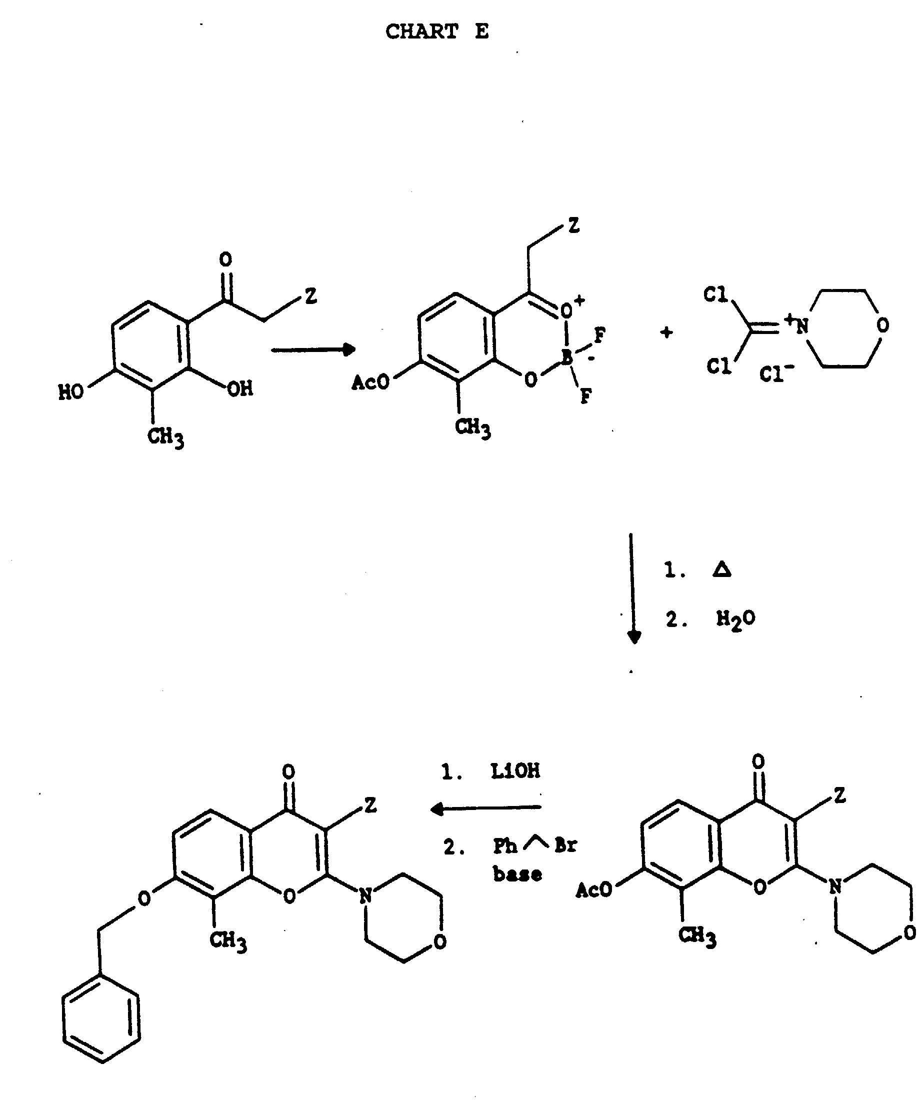

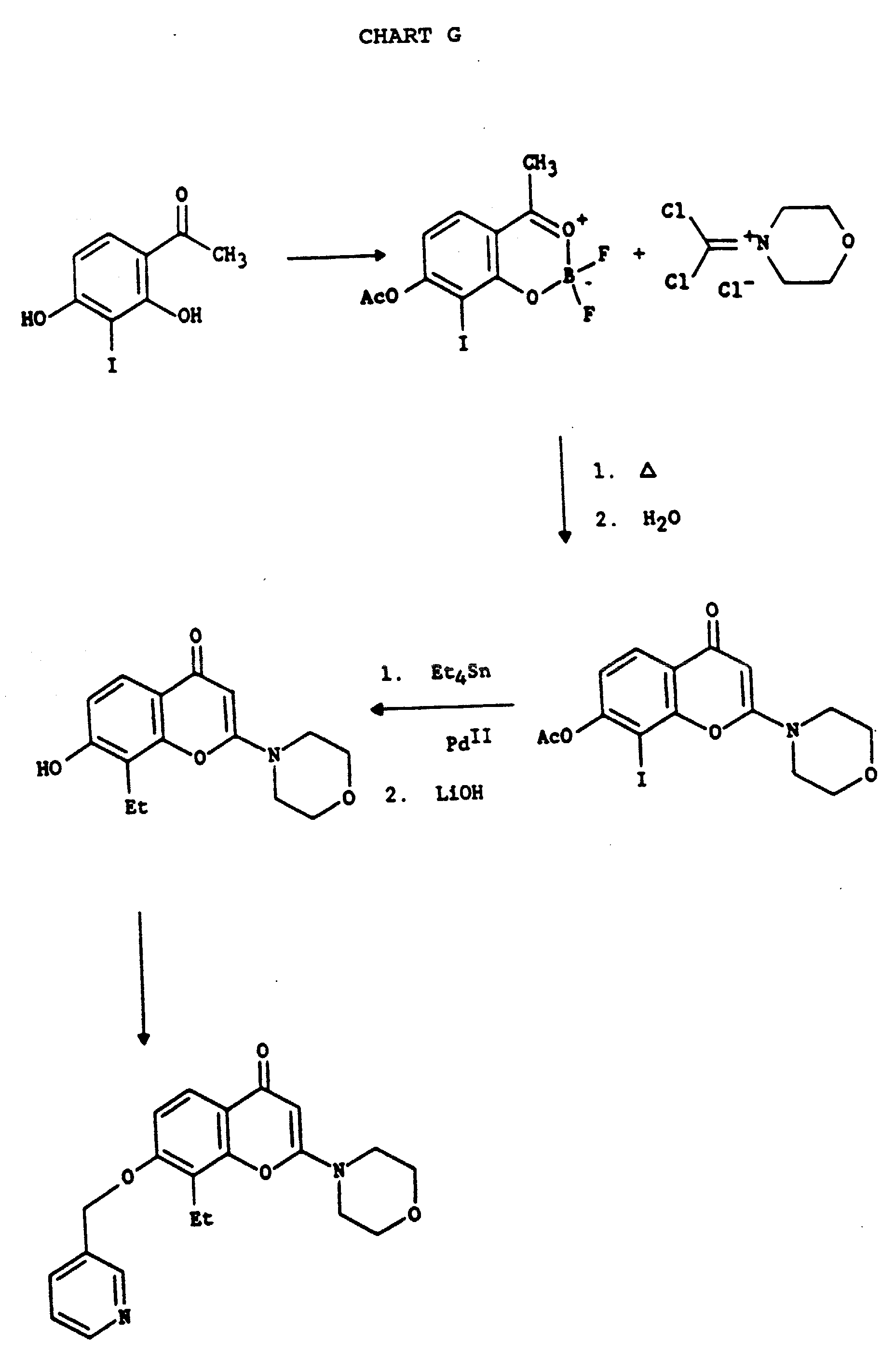

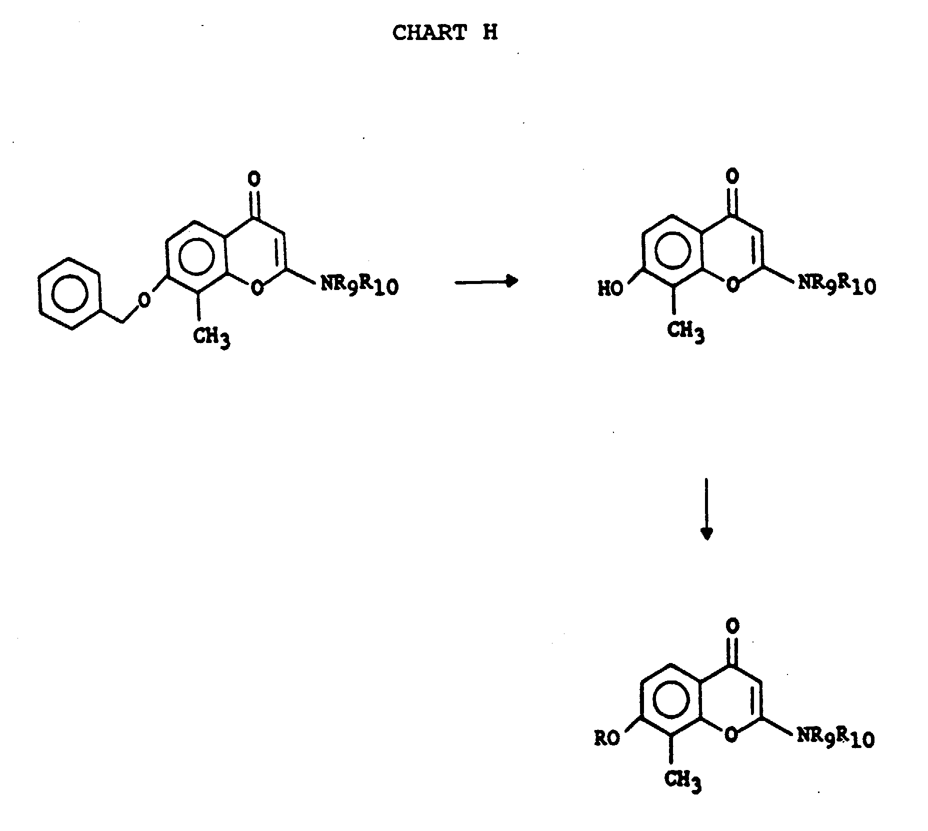

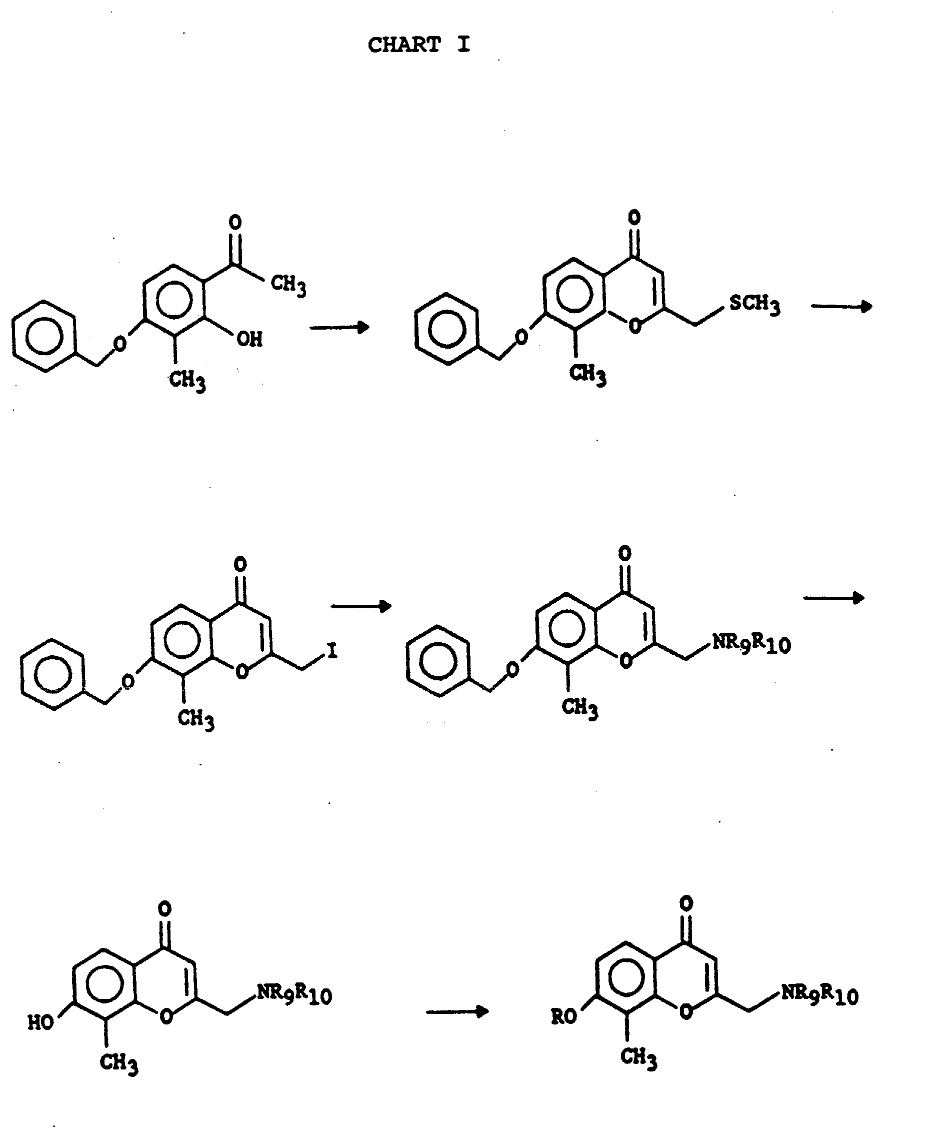

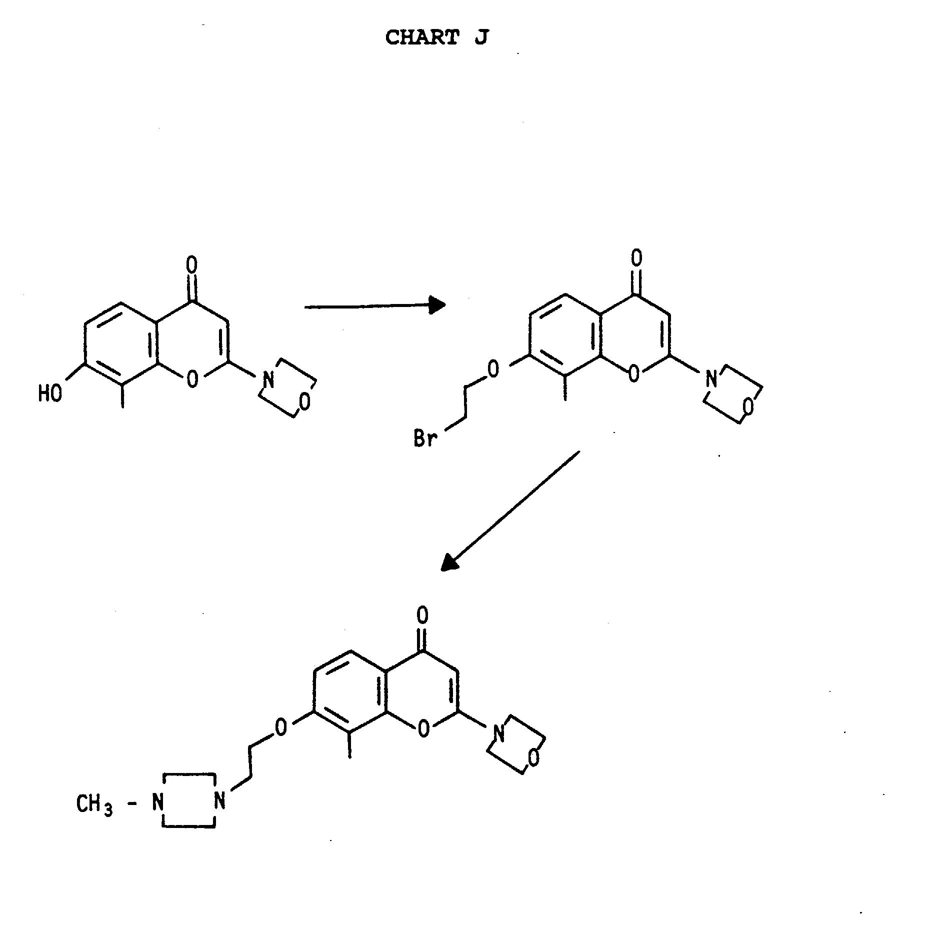

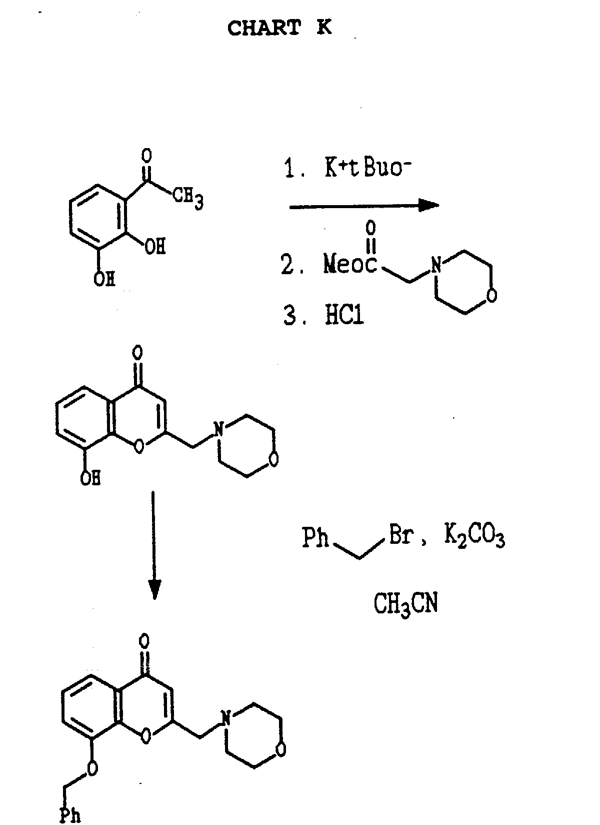

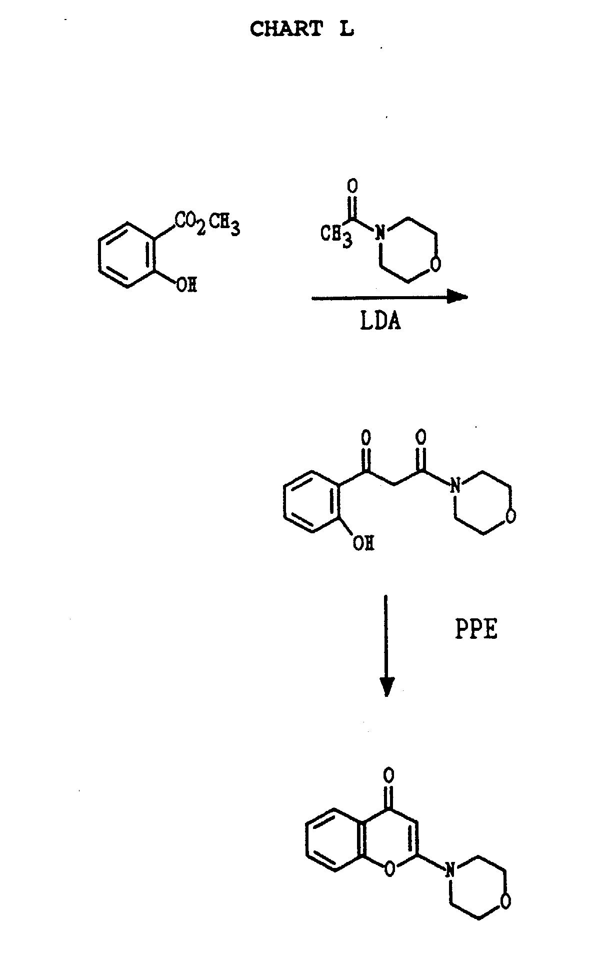

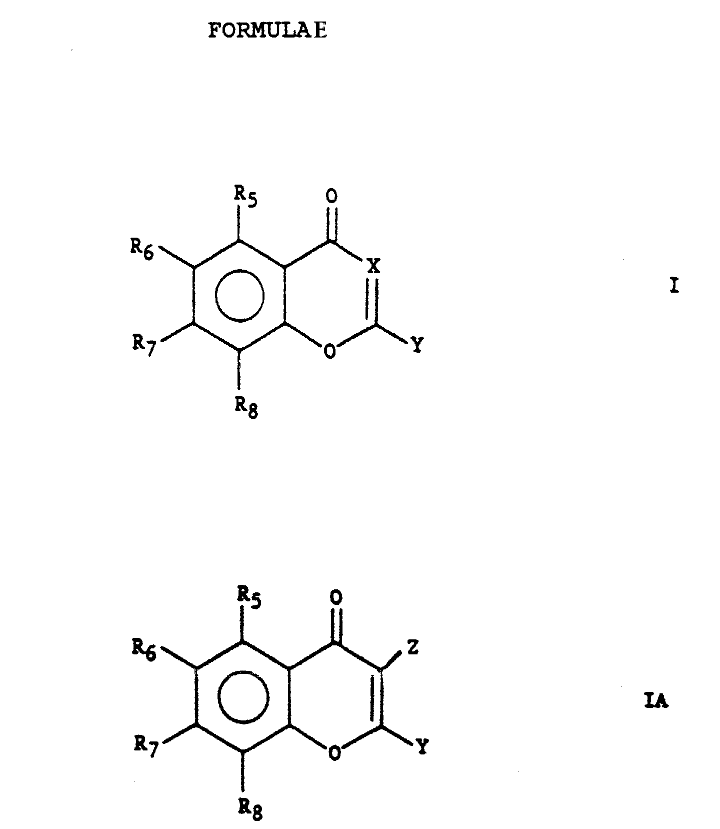

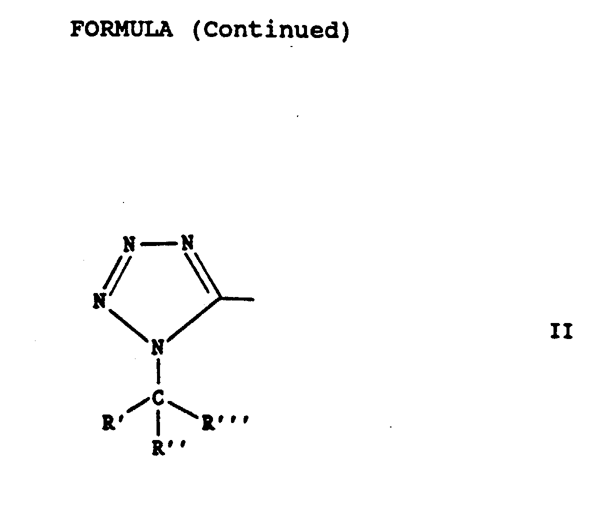

## Claims

1. A compound of Formula I 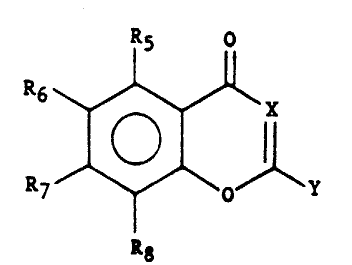 wherein X is CZ where Z is H, C₁-C₅ alkyl, amino (-NH₂) or a halogen atom;
Y is -(CH₂)ₙNR₉R₁₀ wherein R₉ and R₁₀, being the same or different, are selected from (a) hydrogen, with the proviso that R₉ and R₁₀ are not both hydrogen; (b) C₁-C₁₂ alkyl; (c) phenyl optionally substituted with one 2 or 3 C₁-C₄ alkyl, C₁-C₄ alkoxy, halo, OH, trifluoromethyl or -CO₂(C₁-C₄alkyl); (d) -(CH₂)ₙpheny) [wherein phenyl is optionally substituted with one, 2 or 3 C₁-C₄ alkyl, C₁-C₄ alkoxy, halo, OH, trifluoromethyl or -CO₂(C₁-C₄alkyl)]- (e) -(CH₂)ₙpyridinyl and (f) wherein R₉ and R₁₀, taken together with N, form a saturated or unsaturated heterocyclic amine ring selected from
(aa) 4-morpholine optionally substituted with one or two members selected from the group consisting of C₁-C₄ alkyl, C₁-C₄ alkoxy, halo or trifluoromethyl,
(bb) 4-thiomorpholine optionally substituted with one or two members selected from the group consisting of C₁-C₄ alkyl, C₁-C₄ alkoxy, halo or trifluoromethyl,
(cc) 3-amino-1-pyrrolidine,
(dd) 1-pyrrolidine optionally substituted with one or two members selected from the group consisting of C₁-C₄ alkyl, C₁-C₄ alkoxy, halo, OH, -CH₂OH, or trifluoromethyl,
(ee) 1-piperidine optionally substituted with one or two members selected from the group consisting of C₁-C₄ alkyl, C₁-C₄ alkoxy, halo, trifluoromethyl, (CH₂)_{q}OH, CO₂H, -CO₂CH₃, -CO₂CH₂CH₃ or phenyl (wherein phenyl is optionally substituted with one, 2 or 3 C₁-C₄ alkyl, C₁-C₄ alkoxy, halo or trifluoromethyl),
(ff) 1-piperazine, 4-(C₁-C₄alkyl)-1-piperazine, 4-phenyl-1-piperazine (wherein phenyl is optionally substituted with one, 2 or 3 C₁-C₄ alkyl, C₁-C₄ alkoxy, halo or trifluoromethyl) or 4-pyridinyl-1-piperazine optionally substituted with one or two members selected from the group consisting of C₁-C₄ alkyl, C₁-C₄ alkoxy, halo, trifluoromethyl, -CH₂O_{H}, -CO₂H, -CO₂CH₃ or -CO₂CH₂CH₃, and
(gg) thiazolidine, thiazolidine-4-carboxylic acid, pipecolinic acid, p-piperazinacetophenone, 1-homopiperazine, 1-methylhomopiperazine, 4-phenyl-1,2-3,6-tetrahydropyridine, proline, tetrahydrofurylamine,1-(3-hydroxy)pyrrolidine, nipecotamide, 1,2,3,4-tetrahydroisoquinoline or imidazole;
and R₅, R₆, R₇ and R₈, being the same or different, are selected from the group consisting of hydrogen, C₁-C₈ alkyl, -(CH₂)ₙphenyl [wherein phenyl is optionally substituted with one, 2 or 3 C₁-C₄ alkyl, C₁-C₄ alkoxy, halo, OH, trifluoromethyl or -CO₂(C₁-C₄alkyl)], -(CH₂)ₙnaphthyl, -(CH₂)ₙpyridinyl, -(CH₂)_{q}NR₉R₁₀, -CH=CH-phenyl [wherein phenyl is optionally substituted with one, 2 or 3 C₁-C₄ alkyl, C₁-C₄ alkoxy, halo, OH, trifluoromethyl or -CO₂(C₁-C₄alkyl)], -CH₂-CH=CH₂, -CH=CH-CH₃, -CH=CH₂, -O-CH₂-CH=CH₂, -C≡C-phenyl [wherein phenyl is optionally substituted with one, 2 or 3 C₁-C₄ alkyl, C₁-C₄ alkoxy, halo, OH, trifluoromethyl or -CO₂(C₁-C₄alkyl)], -O(CH₂)ₚ(N-methylpiperidin-3-yl), -O-(CH₂)ₚNR₉R₁₀, -O-CH₂CH₍OCH₃)₂, -O-(CH₂)ₚOR₁₅, -O-(CH₂)ₚ-O-(CH₂)ₚ-OR₁₅, -O-(CH₂)ₚ-S-R₁₅, -O-(CH₂)ₚ-O-(CH₂)ₚNR₉R₁₀, -O-(CH₂)ₚ-S-(CH₂)ₚNR₉R₁₀, -O-(CH₂)ₚ-S-(CH₂)ₚ-OR₁₅, -O-(CH₂)ₚ-S(O)-R₁₅, -O-(CH₂)ₚ-S(O₂)-R₁₅, -O-(CH₂)ₚ-S(O)-(CH₂)ₚNR₉R₁₀, -O-(CH₂)ₚ-S(O)-(CH₂)ₚ-OR₁₅, -O-(CH₂)ₚ-S(O₂)-(CH₂)ₚNR₉R₁₀, -O-(CH₂)ₚ-S(O₂)-(CH₂)ₚ-OR₁₅, -O-(CH₂)ₚ-[4-[(CH₂)ₚOR₁₅]-1-piperazine], -O-(CH₂)ₚ-[4-(CH)(phenyl)₂-1-piperazine) [phenyl optionally substituted with one, 2 or 3 C₁-C₄ alkyl, C₁-C₄ alkoxy, halo, OH, trifluoromethyl or -CO₂(C₁-C₄alkyl)], -O-(CH₂)ₚ-[4-(CH₂)_{q}phenyl-1-piperazine] [phenyl optionally substituted with one, 2 or 3 C₁-C₄ alkyl, C₁-C₄ alkoxy, halo, OH, trifluoromethyl or -CO₂(C₁-C₄alkyl)], -O-(CH₂)ₚ-[4-(CH₂)_{q}pyridinyl-1-piperazine] [pyridinyl optionally substituted with one, 2 or 3 C₁-C₄ alkyl, C₁-C₄ alkoxy, halo, OH, trifluoromethyl, NR₉R₁₀ or -CO₂(C₁-C₄alkyl)], -O-(CH₂)ₚ-[4-(NR₉R₁₀ substituted pyridinyl)-1-piperazine, -O-(CH₂)ₚ-(OH substituted 1-piperidine), -O-(CH₂)ₚ-1-pyrrolidin-2-one, -(CH₂)ₙC(O)-(CH₂)ₙR₉, -(CH₂)ₙC(O)O-(CH₂)ₚR₉, -(CH₎ₙC(O)O-(CH₂)ₚNR₉R₁₀, -(CH₂)ₙC(O)(CH₂)ₙNR₉R₁₀, NO₂, -O-(CH₂)ₙC(O)-(CH₂)ₚR₉, -O-(CH₂)ₙC(O)O-(CH₂)ₚR₉, -O-(CH₂)ₙC(O)-(CH₂)ₙNR₉R₁₀, -NR₉R₁₀, -N(R₉)(CH₂)ₙC(O)-(CH₂)ₙR₁₀, -N(R₉)-(CH₂)ₙC(O)O-(CH₂)ₙR₁₀, N(R₉)(CH₂)ₙC(O)-(CH₂)ₙNR₉R₁₀, -O-(CH₂)ₙphenyl [wherein phenyl is optionally substituted with one, 2 or 3 C₁-C₄ alkyl, C₁-C₄ alkoxy, halo, OH, trifluoromethyl or -CO₂(C₁-C₄alkyl)],-O-(CH₂)ₙpyridine, -O(CH₂)ₙC(O)-(CH₂)ₙpyridine, -O-(CH₂)ₙC(O)O-(CH₂)ₙpyridine, -O(CH₂)ₙC(O)-N(R₉)(CH₂)ₙpyridine, -O-(CH₂)ₙquinoxalinyl, -O-(CH₂)ₙquinolinyl, -O-(CH₂)ₙpyrazinyl, -O-(CH₂)ₙnaphthyl,-O-(CH₂)ₙC(O)-(CH₂)ₙnaphthyl, -O-(CH₂)ₙC(O)O-(CH₂)ₙnaphthyl, -O-(CH₂)ₙC(O)NR₉-(CH₂)ₙnaphthyl, halo (fluoro, chloro, bromo, iodo), OH, -(CH₂)_{q}-OH, (CH₂)_{q}OC(O)R₉ -(CH₂)_{q}OC-(O)-NR₉R₁₀, -(1-cyclohexyl-1H-tetrazol-5-yl)C₁-C₄ alkoxy, -[1-(C₁-C₅alkyl)-1H-tetrazol-5-yl]C₁-C₄ alkoxy, -[1-(phenyl)-1H-tetrazol-5-yl]C₁-C₄ alkoxy [wherein phenyl is optionally substituted with one, 2 or 3 C₁-C₄ alkyl, C₁-C₄ alkoxy, halo, OH, trifluoromethyl or -CO₂(C₁-C₄alkyl)], -[1-(pyridinyl)-1H-tetrazol-5-yl]C₁-C₄alkoxy,-[1-(1-phenylethyl)-1H-tetrazol-5-yl]C₁-C₄ alkoxy, -C₁-C₄ alkoxyl, a group of Formula II 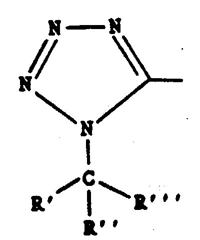
wherein R' is methyl or carboxy, R" is hydrogen and R''' is selected from benzyl [optionally substituted with one, two or three groups selected from hydroxy, halogen or phenoxy (optionally substituted with one, two or three groups selected from hydroxy or halogen)], C₁-C₅ alkyl, -(CH₂)ₙCO₂H, -CH₂SH, -CH₂SCH₃, imidazolinylmethylene, indolinylmethylene, CH₃CH(OH), CH₂OH, H₂N(CH₂)₄-(optionally in protected form) or H₂NC(NH)NH(C-H₂)₃ (optionally in protected form) ; with the overall proviso that at least one member of R₅, R₆, R₇ or R₈ is selected from the group consisting of -CH=CH₂, -O-(CH₂)ₚOH, -O-(CH₂)ₚ-O-(CH₂)ₙpyridin-2-yl,-O-(CH₂)ₚ-O-(CH₂)ₙpyridin-3-yl,-O-(CH₂)ₚ-O-(CH₂)ₙpyridin-4-yl, -O-(CH₂)ₚ-O-(CH₂)ₙ-1-(C₁-C₄alkyl)-1H-5-tetrazole, -O-(CH₂)ₚ-O-(CH₂)ₙ-pyrimidine, -O-(CH₂)ₚ-O-(CH₂)ₙ-2-benzoxazole, -O-(CH₂)ₚ-O-(CH₂)ₙ-2-benzothiazole, -O-(CH₂)ₚ-O-(CH₂)ₙ-(C₁-C₄alkyl)-triazole, -O-(CH₂)ₚ-O-(CH₂)ₙ-(C₁-C₄alkyl)-imidazole, -O-(CH₂)ₚ-O-(CH₂)ₚ-OR₁₅,-O-(CH₂)ₚ-S-R₁₅,-O-(CH₂)ₚ-O-(CH₂)ₚNR₉R₁₀, -O-(CH₂)ₚ-S-(CH₂)ₚNR₉R₁₀, -O-(CH₂)ₚ-S-(CH₂)ₚ-OR₁₅, -O-(CH₂)ₚ-S(O)-R₁₅, -O-(CH₂)ₚ-S(O₂)-R₁₅, -O-(CH₂)ₚ-S(O)-(CH₂)ₚNR₉R₁₀, -O-(CH₂)ₚ-S(O)-(CH₂)ₚ-OR₁₅, -O-(CH₂)ₚ-S(O₂)-(CH₂)ₚNR₉R₁₀, -O-(CH₂)ₚ-S(O₂)-(CH₂)ₚ-OR₁₅, -O-(CH₂)ₚ-[4-[(CH₂)ₚOR₁₅]-1-piperazine], -O-(CH₂)ₚ-[4-(CH)(phenyl)₂-1-piperazine] [phenyl optionally substituted with one, 2 or 3 C₁-C₄ alkyl, C₁-C₄ alkoxy, halo, OH, trifluoromethyl or -CO₂(C₁-c₄alkyl)], -O-(CH₂)ₚ-[4-(CH₂)_{q}phenyl-1-piperazine] [phenyl optionally substituted with one, 2 or 3 C₁-C₄ alkyl, C₁-C₄ alkoxy, halo, OH, trifluoromethyl or -CO₂(C₁-C₄alkyl)], -O-(CH₂)ₚ-[4-(CH₂)_{q}pyridinyl-1-piperazine] [pyridinyl optionally substituted with one, 2 or 3 C₁-C₄ alkyl, C₁-C₄ alkoxy, halo, OH, trifluoromethyl, NR₉R₁₀ or -CO₂(C₁-C₄alkyl)], O-(CH₂)ₚ-[4-(NR₉R₁₀ substituted pyridinyl)-1-piperazine, -O-(CH₂)ₚ-(OH substituted 1-piperidine), -O-(CH₂)ₚ-1-pyrrolidin-2-one;
R₁₅ is selected from H, C₁-C₅ alkyl, -(CH₂)ₙphenyl [phenyl optionally substituted with one, 2 or 3 C₁-C₄ alkyl, C₁-C₄ alkoxy, halo, OH, trifluoromethyl or -CO₂(C₁-C₄alkyl)], -(CH₂)ₙpyridin-1-yl, -(CH₂)ₙpyridin-2-yl, -(CH₂)ₙpyridin-3-yl, -(CH₂)ₙpyridin-4-yl, -CH₂)ₙ-1-(C₁-C₄alkyl)-1H-5-tetrazole, -(CH₂)ₙ-pyrimidine, -(CH₂)ₙ-2-benzoxazole, -(CH₂)ₙ-2-benzothiazole, -(CH₂)ₙ-(C₁-C₄alkyl)-triazole, -(CH₂)ₙ-(C₁-C₄alkyl)-imidazole;
n is 0-5;
p is 2-5;
q is 1-5;
and pharmaceutically acceptable salts and hydrates thereof.

2. A compound according to Claim 1 wherein R₉ and R₁₀ are taken together with N and form a saturated or unsatured heterocyclic amine ring selected from definitions (aa), (bb), (cc), (dd), (ee) and (ff) given in claim 1.

3. A compound according to Claim 1 wherein Z is H or C₁-C₅ alkyl.

4. A compound according to Claim 3 wherein n is 0 or 1, and R₉ and R₁₀, taken together with N, form 4-morpholine.

5. A compound according to Claim 4 wherein Z is H.

6. A compound according to Claim 5 wherein n is 0.

7. A compound according to Claim 2 wherein at least one member selected from R₅, R₆, R₇ or R₈ is _{,} selected from:
-O-(CH₂)ₚ-S-R₁₅, -O-(CH₂)ₚ-[4(CH)phenyl)₂-1-piperazine], -CH=CH₂, or -O-(CH₂)ₚO-(CH₂)ₚOR₁₅.

8. A compound according to Claim 1 selected from the group consisting of:
Cpd 217 2-(4-Morpholinyl)-7-phenylmethoxy-8-vinyl-4H-1-benzopyran-4-one;
Cpd 219 8-Methyl-7-[(2-thiomethyl)ethyl]oxy-2-(4-Morpholinyl)-4H-1-benzopyran-4-one;
Cpd 220 8-Methyl-2-(4-morpholinyl)-7-[2-(4-(2-hydroxy)ethyl-1-piperazinyl)ethyl]oxy-4H-1-benzopyran-4-one;
Cpd 222 7-[2-(Hydroxy)ethyl]oxy-8-methyl-2-(4-morpholinyl)-4H-1-benzopyran-4-one;
Cpd 223 8-Methyl-2-(4-morpholinyl)-7-[2-(2-thiopyridinyl)ethyl]oxy-4H-1-benzopyran-4-one;
Cpd 226 8-Methyl-7-[2-((1-Methyl-1,3-imidazol-2-yl)thio)ethyl]oxy-2-(4-morpholinyl)-4H-1-benzopyran-4-one;
Cpd 228 8-Methyl-7-[2-((4-Methyl-1,2,4-triazol-3-yl)thio)ethyl]oxy-2-(4-morpholinyl)-4H-1-benzopyran-4-one ;
Cpd 230 8-Methyl-7-[2-((1-Methyl-5-tetrazoyl)thio)ethyl]oxy-2-(4-morpholinyl)-4H-1-benzopyran-4-one;
Cpd 231 8-Methyl-2-(4-morpholinyl)-7-[2-((2-pyrimidinyl)thio)ethyl]oxy-4H-1-benzopyran-4-one ;
Cpd 234 7-[2-((2-(Bis-N,N'-diethylamino)ethyl), thio)ethyl]oxy-8-Methyl-2-(4-morpholinyl)-4H-1-benzopyran-4-one;
Cpd 235 8-Methyl-7-[2-((2-benzoxazolyl)thio) ethyl]oxy-2-(4-morpholinyl)-4H-1-benzopyran-4-one;
Cpd 236 8-Methyl-7-[2-((2-benzothiazolyl)thio) ethyl]oxy-2-(4-morpholinyl)-4H-1-benzopyran-4-one;
Cpd 237 7-[2-(4-(3-Ethylamino-pyridin-2-yl)-1-piperazinyl)ethyl]oxy-8-methyl-2-(4-morpholinyl)-4H-1-benzopyran-4-one; and
Cpd 238 8-Methyl-2-(4-morpholinyl)-7-[2-(pyrrolidinone-1-yl)ethyl]oxy-4H-1-benzopyran-4-one ;
or a pharmaceutically-acceptable salt or hydrate thereof.

9. A compound according to Claim 1 selected from the group consisting of:
Cpd 242 2-(4-morpholinyl)-8-[2-(2-pyridinylthio)ethoxy]-4H-1-Benzopyran-4-one;
Cpd 244 8-[2-[4-[3-(ethylamino)-2-pyridinyl]-1-piperazinyl)]ethoxy]-2-(4-morpholinyl)-4H-1-Benzopyran-4-one;
Cpd 246 8-methyl-2-(4-morpholinyl)-7-(2-(phenylsulfinyl)ethoxy] -4H-1-Benzopyran-4-one;
Cpd 253 7-[2-[(2-methoxyphenyl)thio]ethoxy]-8-methyl-2-(4-morpholinyl)-4H-1-Benzopyran-4-one;
Cpd 269 8-methyl-2-(4-morpholinyl)-7-[2-(2-pyridinyloxy)ethoxy)]-4H-1-Benzopyran-4-one;
Cpd 296 2-(4-morpholinyl)-8-[2-[4-(phenylmethyl)-1-piperazinyl]ethoxy]-4H-1-Benzopyran-4-one;
Cpd 326 8-ethenyl-7-[2-(4-methyl-1-piperazinyl)ethoxy]-2-(4-morpholinyl)-4H-1-Benzopyran-4-one;
Cpd 327 8-ethenyl-2-(4-morpholinyl)-7-[2-(1-piperidinyl)ethoxy]-4H-1-Benzopyran-4-one;
Cpd 328 8-ethenyl-1-(4-morpholinyl)-7-[2-(4-phenyl-1-piperidinyl)ethoxy]-4H-1-Benzopyran-4-one;
Cpd 329 8-ethenyl-2-(4-morpholinyl)-7-[2-(1-pyrrolidinyl)ethoxy]-4H-1-Benzopyran-4-one;
Cpd 330 8-ethenyl-2-(4-morpholinyl)-7-[2-(4-thiomorpholinyl)ethoxyl-4H-1-Benzopyran-4-one;
Cpd 331 (R)-8-ethenyl-7-[2-[2-(hydroxymethyl)-1-pyrrolidinyl]ethoxy]-2-(4-morpholinyl)-4H-l-Benzopyran-4-one;
Cpd 333 7-[2-(2-methoxyethoxy)ethoxy]-8-methyl-2-(4-morpholinyl)-4H-1-Benzopyran-4-one;and
Cpd 335 8-methyl-2-(4-morpholinyl)-7-(2-(phenylthio)ethoxy]-4H-1-Benzopyran-4-one;
or a pharmaceutically-acceptable salt or hydrate thereof.

10. A compound which is
Cpd 227 7-[2-((bis-N,N'-(2-methoxy)ethoxy)amino)-ethyl]oxy-8-methyl-2-(4-morpholinyl)-4H-1-benzopyran-4-one; or
Cpd 229 7-[2-(N-ethyl-N'-((2-hydroxy)ethyl)amino)-ethyl]oxy-8-methyl-2-(4-morpholinyl)-4H-1-benzopyran-4-one;
or a pharmaceutically-acceptable salt or hydrate thereof.

11. 7-[2-(4-Ethyl-1-piperazinyl)ethyl]oxy-8-methyl-2-(4-morpholinyl)-4H-1-benzopyran-4-one or a pharmaceutically-acceptable salt thereof.

12. A method for preparing a pharmaceutical composition, which comprises formulating a compound according to any of claims 1 to 11 with a pharmaceutically-acceptable carrier.

13. A process for the preparation of a compound according to any of claims 1 to 11, which comprises reacting a salicyclic acid ester of Formula A 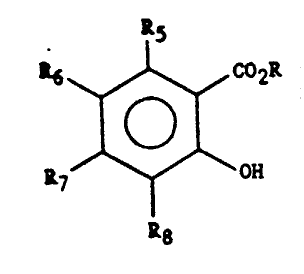 with an ynamine of the formula HC≡C-NR₉R₁₀.

14. Use of a compound according to any of claims 1 to 11, for the manufacture of a medicament for use in preventing or treating atherosclerosis.

15. Use according to claim 14, wherein R₉ and R₁₀ are as defined in claim 2.

## Patentansprüche

1. Verbindung der Formel I 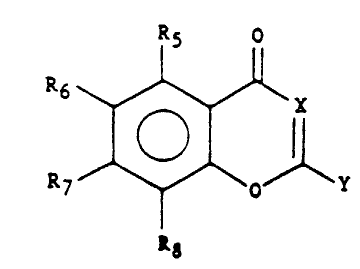 worin bedeuten:
X CZ mit Z gleich H, C₁-C₅-Alkyl, Amino (-NH₂) oder einem Halogenatom;
Y -(CH₂)ₙNR₉R₁₀ mit R₉ und R₁₀, die gleich oder verschieden sind, ausgewählt aus (a) Wasserstoff, wobei gilt, daß R₉ und R₁₀ nicht gleichzeitig Wasserstoff sein dürfen; (b) C₁-C₁₂-Alkyl; (c) Phenyl, gegebenenfalls substituiert durch 1, 2 oder 3 C₁-C₄-Alkyl, C₁-C₄-Alkoxy, Halogen, OH, Trifluormethyl oder -CO₂(C₁-C₄-Alkyl); (d) -(CH₂)ₙPhenyl, wobei Phenyl gegebenenfalls substituiert ist durch 1, 2 oder 3 C₁-C₄-Alkyl, C₁-C₄-Alkoxy, Halogen, OH, Trifluormethyl oder -CO₂(C₁-C₄-Alkyl); (e) -(CH₂)ₙPyridinyl und (f), worin R₉ und R₁₀ zusammen mit N einen gesättigten oder ungesättigten heterocyclischen Aminring, ausgewählt aus
(aa) 4-Morpholin, gegebenenfalls substituiert durch ein oder zwei Glied(er) aus der Gruppe C₁-C₄-Alkyl, C₁-C₄-Alkoxy, Halogen oder Trifluormethyl,
(bb) 4-Thiomorpholin, gegebenenfalls substituiert durch ein oder zwei Glied(er) aus der Gruppe C₁-C₄-Alkyl, C₁-C₄-Alkoxy, Halogen oder Trifluormethyl,
(cc) 3-Amino-1-pyrrolidin,
(dd) 1-Pyrrolidin, gegebenenfalls substituiert durch ein oder zwei Glied(er) aus der Gruppe C₁-C₄-Alkyl, C₁-C₄-Alkoxy, Halogen, OH, -CH₂OH oder Trifluormethyl,
(ee) 1-Piperidin, gegebenenfalls substituiert durch ein oder zwei Glied(er) aus der Gruppe C₁-C₄-Alkyl, C₁-C₄-Alkoxy, Halogen, Trifluormethyl, -(CH₂)_{q}OH, -CO₂H, -CO₂CH₃, -CO₂CH₂CH₃ oder Phenyl, wobei Phenyl gegebenenfalls substituiert ist durch 1, 2 oder 3 C₁-C₄-Alkyl, C₁-C₄-Alkoxy, Halogen oder Trifluormethyl,
(ff) 1-Piperazin, 4-(C₁-C₄-Alkyl)-1-piperazin, 4-Phenyl-1-piperazin, wobei Phenyl gegebenenfalls substituiert ist durch 1, 2 oder 3 C₁-C₄-Alkyl, C₁-C₄-Alkoxy, Halogen oder Trifluormethyl, oder 4-Pyridinyl-1-piperazin, gegebenenfalls substituiert durch eins oder zwei Glied(er) aus der Gruppe C₁-C₄-Alkyl, C₁-C₄-Alkoxy, Halogen, Trifluormethyl, -CH₂OH, -CO₂H, -CO₂CH₃ oder -CO₂CH₂CH₃ und
(gg) Thiazolidin, Thiazolidin-4-carbonsäure, Pipecolinsäure, p-Piperazinacetophenon, 1-Homopiperazin, 1-Methylhomopiperazin, 4-Phenyl-1,2,3,6-Tetrahydropyridin, Prolin, Tetrahydrofurylamin, 1-(3-Hydroxy)pyrrolidin, Nipecotamid, 1,2,3,4-Tetrahydroisochinolin oder Imidazol, bilden,
R₅, R₆, R₇ und R₈, die gleich oder verschieden sind, Wasserstoff, C₁-C₈-Alkyl, -(CH₂)ₙPhenyl [wobei Phenyl gegebenenfalls substituiert ist durch 1, 2 oder 3 C₁-C₄-Alkyl, C₁-C₄-Alkoxy, Halogen, OH, Trifluormethyl oder -CO₂(C₁-C₄-Alkyl)], -(CH₂)ₙNaphthyl, -(CH₂)ₙPyridinyl, -(CH₂)_{q}NR₉R₁₀, -CH=CH-Phenyl [wobei Phenyl gegebenenfalls substituiert ist durch 1, 2 oder 3 C₁-C₄-Alkyl, C₁-C₄-Alkoxy, Halogen, OH, Trifluormethyl oder -CO₂(C₁-C₄-Alkyl)], -CH₂-CH=CH₂, -CH=CH-CH₃, -CH=CH₂, -O-CH₂-CH=CH₂, -C≡C-Phenyl [wobei Phenyl gegebenenfalls substituiert ist durch 1, 2 oder 3 C₁-C₄-Alkyl, C₁-C₄-Alkoxy, Halogen, OH, Trifluormethyl oder -CO₂(C₁-C₄-Alkyl)] , -O(CH₂)ₚ(N-Methylpiperidin-3-yl), -O-(CH₂)ₚNR₉R₁₀, -O-CH₂CH(OCH₃)₂, -O-(CH₂)ₚOR₁₅, -O-(CH₂)ₚ-O-(CH₂)ₚ-OR₁₅, -O-(CH₂)ₚ-S-R₁₅, -O-(CH₂)ₚ-O-(CH₂)ₚNR₉R₁₀, -O-(CH₂)ₚ-S-(CH₂)ₚNR₉R₁₀, -O-(CH₂)ₚ-S-(CH₂)ₚ-OR₁₅, -O-(CH₂)ₚ-S(O)-R₁₅, -O-(CH₂)ₚ-S(O₂)-R₁₅, -O-(CH₂)ₚ-S(O)-(CH₂)ₚNR₉R₁₀, -O-(CH₂)ₚ-S(O)-(CH₂)ₚ-OR₁₅, -O-(CH₂)ₚ-S(O₂)-(CH₂)ₚNR₉R₁₀, -O-(CH₂)ₚ-S(O₂)-(CH₂)ₚ-OR₁₅, -O-(CH₂)ₚ-[4-[(CH₂)ₚOR₁₅]-1-Piperazin, -O-(CH₂)ₚ-[4-(CH)(Phenyl)₂-1-Piperazin] [wobei Phenyl gegebenenfalls substituiert ist durch 1, 2 oder 3 C₁-C₄-Alkyl, C₁-C₄-Alkoxy, Halogen, OH, Trifluormethyl oder -CO₂(C₁-C₄-Alkyl)], -O-(CH₂)ₚ-[4-(CH₂)_{q}Phenyl-1-piperazin [wobei Phenyl gegebenenfalls substituiert ist durch 1, 2 oder 3 C₁-C₄-Alkyl, C₁-C₄-Alkoxy, Halogen, OH, Trifluormethyl oder -CO₂(C₁-C₄-Alkyl)], -O-(CH₂)ₚ-[4-(CH₂)_{q}-Pyridinyl-1-piperazin [wobei Pyridinyl gegebenenfalls substituiert ist 1, 2 oder 3 C₁-C₄-Alkyl, C₁-C₄-Alkoxy, Halogen, OH, Trifluormethyl oder NR₉R₁₀ oder -CO₂(C₁-C₄-Alkyl)], -O-(CH₂)ₚ-[4-(NR₉R₁₀ substituiertes Pyridinyl)-1-piperazin, -O-(CH₂)ₚ-(OH substituiertes 1-Piperidin), -O-(CH₂)ₚ-1-Pyrrolidin-2-on, -(CH₂)ₙC(O)-(CH₂)ₙR₉, -(CH₂)ₙC(O)O-(CH₂)ₚR₉, -(CH₂)ₙC(O)O-(CH₂)ₚNR₉R₁₀, -(CH₂)ₙC(O)-(CH₂)ₙNR₉R₁₀, NO₂, -O-(CH₂)ₙC(O)-(CH₂)ₚR₉, -O-(CH₂)ₙC(O)O-(CH₂)ₚR₉, -O-(CH₂)ₙC(O)-(CH₂)ₙNR₉R₁₀, -NR₉R₁₀, -N(R₉)(CH₂)ₙC(O)-(CH₂)ₙR₁₀, -N(R₉)-(CH₂)ₙC(O)O-(CH₂)ₙR₁₀, N(R₉)(CH₂)ₙC(O)-(CH₂)ₙNR₉R₁₀, O-(CH₂)ₙPhenyl [wobei Phenyl gegebenenfalls substituiert ist durch 1, 2 oder 3 C₁-C₄-Alkyl, C₁-C₄-Alkoxy, Halogen, OH, Trifluormethyl oder -CO₂(C₁-C₄-Alkyl)], -O-(CH₂)ₙPyridin, -O(CH₂)ₙC(O)-(CH₂)ₙPyridin, -O-(CH₂)ₙC(O)O-(CH₂)ₙPyridin, -O(CH₂)ₙC(O)-N(R₉)(CH₂)ₙPyridin, -O-(CH₂)ₙChinoxalinyl, -O-(CH₂)ₙChinolinyl, -O-(CH₂)ₙPyrazinyl, -O-(CH₂)ₙNaphthyl, -O-(CH₂)ₙC(O)-(CH₂)ₙNaphthyl, -O-(CH₂)ₙC(O)O-(CH₂)ₙNaphthyl, -O-(CH₂)ₙC(O)NR₉-(CH₂)ₙNaphthyl, Halogen (Fluor, Chlor, Brom, Iod), OH, -(CH₂)_{q}-OH, (CH₂)_{q}OC(O)R₉, -(CH₂)_{q}OC-(O)-NR₉R₁₀, -(1-Cyclohexyl-1H-tetrazol-5-yl)C₁-C₄-Alkoxy, -[1-(C₁-C₅-Alkyl)-1H-tetrazol-5-yl]C₁-C₄-Alkoxy, -[1-(Phenyl)-1H-tetrazol-5-yl]C₁-C₄-Alkoxy [wobei Phenyl gegebenenfalls substituiert ist durch 1, 2 oder 3 C₁-C₄-Alkyl, C₁-C₄-Alkoxy, Halogen, OH, Trifluormethyl oder -CO₂(C₁-C₄-Alkyl)], -[1-(Pyridinyl)-1H-tetrazol-5-yl]C₁-C₄-Alkoxy, -[1-(1-Phenylethyl)-1H-tetrazol-5-yl]C₁-C₄-Alkoxy, -C₁-C₄-Alkoxy, eine Gruppe der Formel II 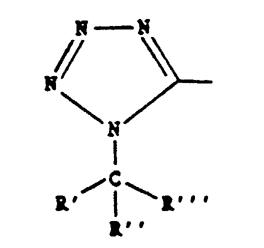 worin bedeuten:
R' Methyl oder Carboxy;
R'' Wasserstoff und
R''' Benzyl [gegebenenfalls substituiert durch 1, 2 oder 3 Gruppen, ausgewählt aus Hydroxy, Halogen oder Phenoxy (gegebenenfalls substituiert durch 1, 2 oder 3 Gruppen, ausgewählt aus Hydroxy oder Halogen)], C₁-C₅-Alkyl, -(CH₂)ₙCO₂H, -CH₂SH, -CH₂SCH₃, Imidazolinylmethylen, Indolinylmethylen, CH₃CH(OH), CH₂OH, H₂N(CH₂)4-(gegebenenfalls in geschützter Form) oder H₂NC(NH)NH(CH₂)₃ (gegebenenfalls in geschützter Form);
wobei insgesamt gilt, daß mindestens einer der Reste R₅, R₆, R₇ oder R₈ aus der Gruppe -CH=CH₂, -O-(CH₂)ₚOH, -O-(CH₂)ₚ-O-(CH₂)ₙPyridin-2-yl, -O-(CH₂)ₚ-O-(CH₂)ₙPyridin-3-yl, -O-(CH₂)ₚ-O-(CH₂)ₙPyridin-4-yl, -O-(CH₂)ₚ-O-(CH₂)ₙ-1-(C₁-C₄-Alkyl)-1H-5-tetrazol, -O-(CH₂)ₚ-O-(CH₂)ₙ-Pyrimidin, -O-(CH₂)ₚ-O-(CH₂)ₙ-2-Benzoxazol, -O-(CH₂)ₚ-O-(CH₂)ₙ-2-Benzothiazol, -O-(CH₂)ₚ-O-(CH₂)ₙ-(C₁-C₄-Alkyl)-triazol, -O-(CH₂)ₚ-O-(CH₂)ₙ-(C₁-C₄-Alkyl)-imidazol, -O-(CH₂)ₚ-O-(CH₂)ₚ-OR₁₅, -O-(CH₂)ₚ-S-R₁₅, -O-(CH₂)ₚ-O-(CH₂)ₚNR₉R₁₀, -O-(CH₂)ₚ-S-(CH₂)ₚNR₉R₁₀, -O-(CH₂)ₚ-S-(CH₂)ₚ-OR₁₅, -O-(CH₂)ₚ-S(O)-R₁₅, -O-(CH₂)ₚ-S(O₂)-R₁₅, -O-(CH₂)ₚ-S(O)-(CH₂)ₚNR₉R₁₀, -O-(CH₂)ₚ-S(O)-(CH₂)ₚ-OR₁₅, -O-(CH₂)ₚ-S(O₂)-(CH₂)ₚNR₉R₁₀, -O-(CH₂)ₚ-S(O₂)-(CH₂)ₚ-OR₁₅, -O-(CH₂)ₚ-[4-[(CH₂)ₚOR₁₅]-1-Piperazin], -O-(CH₂)ₚ-[4-(CH)(Phenyl)₂-1-piperazin] [wobei Phenyl gegebenenfalls substituiert ist durch 1, 2 oder 3 C₁-C₄-Alkyl, C₁-C₄-Alkoxy, Halogen, OH, Trifluormethyl oder -CO₂(C₁-C₄-Alkyl)], -O-(CH₂)ₚ-[4-(CH₂)_{q}-Phenyl-1-piperazin [wobei Phenyl gegebenenfalls substituiert ist durch 1, 2 oder 3 C₁-C₄-Alkyl, C₁-C₄-Alkoxy, Halogen, OH, Trifluormethyl oder -CO₂(C₁-C₄-Alkyl)], -O-(CH₂)ₚ-[4-(CH₂)_{q}Pyridinyl-1-piperazin [wobei Pyridinyl gegebenenfalls substituiert ist durch 1, 2 oder 3 C₁-C₄-Alkyl, C₁-C₄-Alkoxy, Halogen, OH, Trifluormethyl, NR₉R₁₀ oder -CO₂ (C₁-C₄-Alkyl)], O-(CH₂)ₚ-[4-(NR₉R₁₀ substituiertes Pyridinyl)-1-piperazin, -O-(CH₂)ₚ-(OH substituiertes 1-Piperidin), -O-(CH₂)ₚ-1-Pyrrolidin-2-on ausgewählt ist und R₁₅ aus H, C₁-C₅-Alkyl, -(CH₂)ₙPhenyl [wobei Phenyl gegebenenfalls substituiert ist durch 1, 2 oder 3 C₁-C₄-Alkyl, C₁-C₄-Alkoxy, Halogen, OH, Trifluormethyl oder -CO₂(C₁-C₄-Alkyl)], -(CH₂)ₙPyridin-1-yl, -(CH₂)ₙPyridin-2-yl, -(CH₂)ₙPyridin-3-yl, -(CH₂)ₙPyridin-4-yl, -(CH₂)ₙ-1-(C₁-C₄-Alkyl)-1H-5-tetrazol, -(CH₂)ₙ-Pyrimidin, -(CH₂)ₙ-2-Benzoxazol, -(CH₂)ₙ-2-Benzothiazol, -(CH₂)ₙ-(C₁-C₄-Alkyl)-triazol, -(CH₂)ₙ-(C₁-C₄-Alkyl)-imidazol ausgewählt ist und
n = 0-5;
p = 2-5;
q = 1-5; und
pharmazeutisch akzeptable Salze und Hydrate hiervon.

2. Verbindung nach Anspruch 1, wobei R₉ und R₁₀ zusammen mit dem N einen gesättigten oder ungesättigten heterocyclischen Aminring, ausgewählt aus den Definitionen (aa), (bb), (cc), (dd), (ee) und (ff) in Anspruch 1 bilden.

3. Verbindung nach Anspruch 1, wobei Z für H oder C₁-C₅-Alkyl steht.

4. Verbindung nach Anspruch 3, wobei n = 0 oder 1 und R₉ und R₁₀ zusammen mit N 4-Morpholin bilden.

5. Verbindung nach Anspruch 4, wobei Z für H steht.

6. Verbindung nach Anspruch 5, wobei n = 0.

7. Verbindung nach Anspruch 2, wobei mindestens einer der Reste R₅, R₆, R₇ oder R₈ aus -O-(CH₂)ₚ-S-R₁₅, -O-(CH₂)ₚ-[4-(CH)Phenyl)₂-1-piperazin], -CH=CH₂ oder -O-(CH₂)ₚO-(CH₂)ₚOR₁₅ ausgewählt ist.

8. Verbindung nach Anspruch 1, ausgewählt aus der Gruppe:
Cpd 217 2-(4-Morpholinyl)-7-phenylmethoxy-8-vinyl-4H-1-benzopyran-4-on;
Cpd 219 8-Methyl-7-[(2-thiomethyl)ethyl]oxy-2-(4-morpholinyl)-4H-1-benzopyran-4-on;
Cpd 220 8-Methyl-2-(4-morpholinyl)-7-[2-(4-(2-hydroxy)ethyl-1-piperazinyl)ethyl]oxy-4H-1-benzopyran-4-on;
Cpd 222 7-[2-(Hydroxy)ethyl]oxy-8-methyl-2-(4-morpholinyl)-4H-1-benzopyran-4-on;
Cpd 223 8-Methyl-2-(4-morpholinyl)-7-[2-(2-thiopyridinyl)ethyl]oxy-4H-1-benzopyran-4-on;
Cpd 226 8-Methyl-7-[2-((1-methyl-1,3-imidazol-2-yl)thio)ethyl]oxy-2-(4-morpholinyl)-4H-1-benzopyran-4-on;
Cpd 228 8-Methyl-7-[2-((4-methyl-1,2,4-triazol-3-yl)thio)ethyl]oxy-2-(4-morpholinyl)-4H-1-benzopyran-4-on;
Cpd 230 8-Methyl-7-[2-((1-methyl-5-tetrazoyl)thio)ethyl]oxy-2-(4-morpholinyl)-4H-1-benzopyran-4-on;
Cpd 231 8-Methyl-2-(4-morpholinyl)-7-[2-((2-pyrimidinyl)thio)ethyl]oxy-4H-1-benzopyran-4-on;
Cpd 234 7-[2-((2-(Bis-N,N'-diethylamino)ethyl)thio)ethyl]oxy-8-methyl-2-(4-morpholinyl)-4H-1-benzopyran-4-on;
Cpd 235 8-Methyl-7-[2-((2-benzoxazolyl)thio)ethyl]oxy-2-(4-morpholinyl)-4H-1-benzopyran-4-on;
Cpd 236 8-Methyl-7-[2-((2-Benzothiazolyl)thio)ethyl]oxy-2-(4-morpholinyl)-4H-1-benzopyran-4-on;
Cpd 237 7-[2-(4-(3-Ethylamino-pyridin-2-yl)-1-piperazinyl)ethyl]oxy-8-methyl-2-(4-morpholinyl)-4H-1-benzopyran-4-on und
Cpd 238 8-Methyl-2-(4-morpholinyl) -7-[2-(pyrrolidinon-1-yl)ethyl]oxy-4H-1-benzopyran-4-on,
oder ein pharmazeutisch akzeptables Salz oder Hydrat hiervon.

9. Verbindung nach Anspruch 1, ausgewählt aus der Gruppe:
Cpd 242 2-(4-Morpholinyl)-8-[2-(2-pyridinylthio)ethoxy]-4H-1-benzopyran-4-on;
Cpd 244 8-[2-[4-[3-(Ethylamino) -2-pyridinyl]-1-piperazinyl]ethoxy]-2-(4-morpholinyl)-4H-1-benzopyran-4-on;
Cpd 246 8-Methyl-2-(4-morpholinyl)-7-[2-(phenylsulfinyl)ethoxy]-4H-1-benzopyran-4-on;
Cpd 253 7-[2-[(2-Methoxyphenyl)thio]ethoxy]-8-methyl-2-(4-morpholinyl) -4H-1-benzopyran-4-on;
Cpd 269 8-Methyl-2-(4-morpholinyl)-7-[2-(2-pyridinyloxy)ethoxy]-4H-1-benzopyran-4-on;
Cpd 296 2-(4-Morpholinyl)-8-[2-[4-(phenylmethyl)-1-piperazinyl]ethoxy]-4H-1-benzopyran-4-on;
Cpd 326 8-Ethenyl-7-[2-(4-methyl-1-piperazinyl)ethoxy]-2-(4-morpholinyl)-4H-1-benzopyran-4-on;
Cpp 327 8-Ethenyl-2-(4-morpholinyl)-7-[2-(1-piperidinyl)ethoxy]-4H-1-benzopyran-4-on;
Cpd 328 8-Ethenyl-l-(4-morpholinyl)-7-[2-(4-phenyl-1-piperidinyl)ethoxy]-4H-1-benzopyran-4-on;
Cpd 329 8-Ethenyl-2-(4-morpholinyl)-7-[2-(1-pyrrolidinyl)ethoxy]-4H-1-benzopyran-4-on;
Cpd 330 8-Ethenyl-2-(4-morpholinyl)-7-[2-(4-thiomorpholinyl)ethoxy]-4H-1-benzopyran-4-on;
Cpd 331 (R)-8-Ethenyl-7-[2-[2-(hydroxymethyl)-1-pyrrolidinyl]ethoxy]-2-(4-morpholinyl)-4H-1-benzopyran-4-on;
Cpd 333 7-[2-(2-Methoxyethoxy)ethoxy]-8-methyl-2-(4-morpholinyl)-4H-1-benzopyran-4-on und
Cpd 335 8-Methyl-2-(4-morpholinyl)-7-[2-(phenylthio)ethoxy]-4H-1-benzopyran-4-on,
oder ein pharmazeutisch akzeptables Salz oder Hydrat hiervon.

10. Verbindung, nämlich
Cpd 227 7-[2-((Bis-N,N'-(2-methoxy)ethoxy)amino)ethyl]oxy-8-methyl-2-(4-morpholinyl)-4H-1-benzopyran-4-on oder
Cpd 229 7-[2-(N-Ethyl-N'-((2-hydroxy)ethyl)amino)ethyl]oxy-8-methyl-2-(4-morpholinyl)-4H-1-benzopyran-4-on,
oder ein pharmazeutisch akzeptables Salz oder Hydrat hiervon.

11. 7-[2-(4-Ethyl-1-piperazinyl)ethyl]oxy-8-methyl-2-(4-morpholinyl)-4H-1-benzopyran-4-on oder ein pharmazeutisch akzeptables Salz hiervon.

12. Verfahren zur Herstellung einer pharmazeutischen Zubereitung durch Formulieren einer Verbindung nach einem der Ansprüche 1 bis 11 mit einem pharmazeutisch akzeptablen Träger.

13. Verfahren zur Herstellung einer Verbindung nach einem der Ansprüche 1 bis 11 durch Umsetzen eines Salicylsäureesters der Formel A 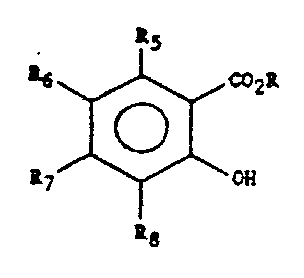 mit einem Inamin der Formel HC≡C-NR₉R₁₀.

14. Verwendung einer Verbindung nach einem der Ansprüche 1 bis 11 zur Herstellung eines Medikaments zur Verwendung bei der Verhinderung oder Behandlung von Atherosklerose.

15. Verwendung nach Anspruch 14, wobei R₉ und R₁₀ die in Anspruch 2 angegebene Bedeutung besitzen.

## Revendications

1. Composé de formule I 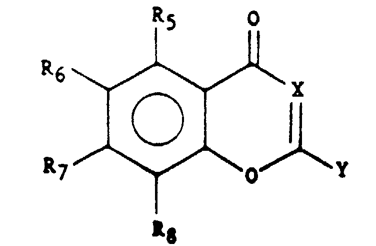 dans laquelle X représente un groupe CZ dans lequel Z représente H, un groupe alkyle en C₁ à C₅, amino (-NH₂) ou un atome d'halogène ;
Y représente un groupe de formule
-(CH₂)ₙNR₉R₁₀ dans laquelle R₉ et R₁₀, identiques ou différents, sont choisis entre (a) l'hydrogène, sous réserve que R₉ et R₁₀ ne représentent pas l'un et l'autre l'hydrogène ; (b) des groupes alkyle en C₁ à C₁₂ ; (c) un groupe phényle facultativement substitué avec 1, 2 ou 3 groupes alkyle en C₁ à C₄, alkoxy en C₁ à C₄, halogéno, OH, trifluorométhyle ou -CO₂(alkyle en C₁ à C₄) ; (d) des groupes -CH₂)ₙphényle [dans lesquels le groupe phényle est facultativement substitué avec 1, 2 ou 3 groupes alkyle en C₁ à C₄, alkoxy en C₁ à C₄, halogéno, OH, trifluorométhyle ou -CO₂(alkyle en C₁ à C₄)], (e) des groupes -(CH₂)ₙpyridinyle, et dans laquelle (f) R₉ et R₁₀, pris conjointement avec N, forment un noyau amine hétérocyclique saturé ou insaturé choisi entre
(aa) la 4-morpholine facultativement substituée avec un ou deux représentants du groupe consistant en groupes alkyle en C₁ à C₄, alkoxy en C₁ à C₄, halogéno et trifluorométhyle,
(bb) la 4-thiomorpholine facultativement substituée avec un ou deux représentants du groupe consistant en groupes alkyle en C₁ à C₄, alkoxy en C₁ à C₄, halogéno et trifluorométhyle,
(cc) la 3-amino-1-pyrrolidine,
(dd) la 1-pyrrolidine facultativement substituée avec un ou deux représentants du groupe consistant en groupes alkyle en C₁ à C₄, alkoxy en C₁ à C₄, halogéno, OH, -CH₂OH et trifluorométhyle,
(ee) la 1-pipéridine facultativement substituée avec un ou deux représentants du groupe consistant en groupes alkyle en C₁ à C₄, alkoxy en C₁ à C₄, halogéno, trifluorométhyle, -(CH₂)_{q}OH, -CO₂H,-CO₂CH₂, -CO₂CH₂CH₃ et phényle (le groupe phényle étant facultativement substitué avec un, 2 ou 3 groupes alkyle en C₁ à C₄, alkoxy en C₁ à C₄, halogéno ou trifluorométhyle),
(ff) la 1-pipérazine, la 4-(alkyle en C₁ à C₄)-1-pipérazine, la 4-phényl-1-pipérazine (dans laquelle le groupe phényle est facultativement substitué avec un, 2 ou 3 groupes alkyle en C₁ à C₄, alkoxy en C₁ à C₄, halogéno ou trifluorométhyle) ou la 4-pyridinyl-1-pipérazine facultativement substituée avec un ou deux représentants du groupe consistant en groupes alkyle en C₁ à C₄, alkoxy en C₁ à C₄, halogéno, trifluorométhyle, -CH₂OH, -CO₂H, -CO₂CH₃ ou -CO₂CH₂CH₃ et
(gg) la thiazolidine, l'acide thiazolidine-4-carboxylique, l'acide pipécolinique, la p-pipérazinacétophénone, la 1-homopipérazine, la 1-méthylhomopipérazine, la 4-phényl-1,2-3,6-tétrahydropyridine, la proline, la tétrahydrofurylamine, la 1-(3-hydroxy)pyrrolidine, le nipécotamide, la 1,2,3,4-tétrahydro-isoquinoléine ou l'imidazole ;
et R₅, R₆, R₇ et R₈, qui sont identiques ou différents, sont choisis dans le groupe consistant en l'hydrogène, des groupes alkyle en C₁ à C₈, -(CH₂)ₙphényle [dans lequel le groupe phényle est facultativement substitué avec un, 2 ou 3 groupes alkyle en C₁ à C₄, alkoxy en C₁ à C₄, halogéno, OH, trifluorométhyle ou -CO₂(alkyle en C₁ à C₄)], -(CH₂)ₙnaphtyle, -(CH₂)ₙpyridinyle, -(CH₂)_{q}NR₉R₁₀, -CH=CH-phényle [dans lequel le groupe phényle est facultativement substitué avec un, 2 ou 3 groupes alkyle en C₁ à C₄, alkoxy en C₁ à C₄, halogéno, OH, trifluorotométhyle ou -CO₂(alkyle en C₁ à C₄)], -CH₂-CH=CH₂, -CH=CH-CH₃, -CH=CH₂, -O-CH₂-CH=CH₂, -C≡C-phényle [dans lequel le groupe phényle est facultativement substitué avec un, 2 ou 3 groupes alkyle en C₁ à C₄, alkoxy en C₁ à C₄, halogéno, OH, trifluorométhyle ou -CO₂ (alkyle en C₁ à C₄)], -O(CH₂)ₚ(N-méthylpipéridine-3-yle), -O-(CH₂)ₚNR₉R₁₀, -O-CH₂CH(OCH₃)₂, -O-(CH₂)ₚOR₁₅, -O-(CH₂)ₚ-OR₁₅, -O-(CH₂)ₚ-S-R₁₅, -O-(CH₂)ₚ-O-(CH₂)ₚNR₉R₁₀, -O-(CH₂)ₚ-S-(CH₂)ₚNR₉R₁₀, -O-(CH₂)ₚ-S-(CH₂)ₚ-OR₁₅, -O-(CH₂)ₚ-S(O)-R₁₅, -O-(CH₂)ₚ-S(O₂)-R₁₅, -O-(CH₂)ₚ-S(O)-(CH₂)ₚNR₉R₁₀, -O-(CH₂)ₚ-S(O)-(CH₂)ₚ-OR₁₅ -O-(CH₂)ₚ-S(O₂)-(CH₂)ₚNR₉R₁₀, -O-(CH₂)ₚ-S(O₂)-(CH₂)ₚ-OR₁₅, -O-(CH₂)ₚ-[4-[(CH₂)ₚOR₁₅]-1-pipérazine], -O(CH₂)ₚ-[4-(CH)) (phényl)₂-1-pipérazine] [le groupe phényle étant facultativement substitué avec un, 2 ou 3 groupes alkyle en C₁ à C₄, alkoxy en C₁ à C₄, halogéno, OH, trifluorométhyle ou -CO₂ (alkyle en C₁ à C₄)] , -O-(CH₂)ₚ-[4-(CH₂)_{q}phényl-1-pipérazine] [le groupe phényle étant facultativement substitué avec un, 2 ou 3 groupes alkyle en C₁ à C₄, alkoxy en C₁ à C₄, halogéno, OH, trifluorométhyle ou -CO₂ (alkyle en C₁ à C₄)] , -O-(CH₂)ₚ-[4-(CH₂)_{q}pyridinyl-1-pipérazine] [le groupe pyridinyle étant facultativement substitué avec un, 2 ou 3 groupes alkyle en C₁ à C₄, alkoxy en C₁ à C₄, halogéno, OH, trifluorométhyle, NR₉R₁₀ ou -CO₂ (alkyle en C₁ à C₄)], -O-(CH₂)ₚ-[4-(pyridinyle à substituant NR₉R₁₀)-1-pipérazine, -O-(CH₂)ₚ-(1-pipéridine à substituant OH), -O-(CH₂)ₚ-1-pyrrolidine-2-one, -(CH₂)ₙC(O)-(CH₂)ₙR₉, -(CH₂)ₙC(O)O-(CH₂)ₚR₉, -(CH₂)ₙC(O)O-(CH₂)ₚNR₉R₁₀, -(CH₂)ₙC(O)(CH₂)ₙNR₉R₁₀, NO₂, -O-(CH₂)ₙC(O)-(CH₂)ₚR₉, -O-(CH₂)ₙC(O)O-(CH₂)ₚR₉, -O-(CH₂)ₙC(O)-(CH₂)ₙNR₉R₁₀, -NR₉R₁₀, -N-(R₉)(CH₂)ₙC(O)-(CH₂)ₙR₁₀, -N(R₉)-(CH₂)ₙC(O)O-(CH₂)ₙR₁₀, N(R₉)(CH₂)ₙC(O)-(CH₂)ₙNR₉R₁₀, -O-(CH₂)ₙphényle [dans lequel le groupe phényle est facultativement substitué avec un, 2 ou 3 groupes alkyle en C₁ à C₄, alkoxy en C₁ à C₄, halogéno, OH, trifluorométhyle ou -CO₂(alkyle en C₁ à C₄)), -O-(CH₂)ₙpyridine, -O(CH₂)ₙC(O)-(CH₂)ₙpyridine, -O-(CH₂)ₙC(O)O-(CH₂)ₙ-pyridine, -O(CH₂)ₙC(O)-N(R₉)(CH₂)ₙpyridine, -O-(CH₂)ₙquinoxalinyle, -O-(CH₂)ₙquinolinyle, -O-(CH₂)ₙpyrazinyle, -O-(CH₂)ₙnaphtyle, -O-(CH₂)ₙC(O)-(CH₂)ₙnapthyle, -O-(CH₂)ₙC(O)O-(CH₂)ₙnaphtyle, -O-(CH₂)ₙC(O)NR₉)-CH₂)ₙnaphtyle, halogéno (fluoro, chloro, bromo, iodo), OH, -(CH₂)_{q}-OH,(CH₂)_{q}OC(O)R₉, -(CH₂)_{q}OC-(O)-NR₉R₁₀, -(1-cyclohexyl-1H-tétrazole-5-yl)-(alkoxy en C₁ à C₄), -[1-(alkyle en C₁ à C₅)-1H-tétrazole-5-yle]-alkoxy en C₁ à C₄), -[1-(phényl)-1H-tétrazole-5-yl]-(alkoxy en C₁ à C₄), [dans lequel le groupe phényle est facultativement substitué avec un, 2 ou 3 groupes alkyle en C₁ à C₄, alkoxy en C₁ à C₄, halogéno, OH, trifluorométhyle ou -CO₂(alkyle en C₁ à C₄)), -([1-pyridinyl) -1H-tétrazole-5-yl]-(alkoxy en C₁ à C₄), -[1-(1-phényléthyl)-1H-tétrazole-5-yl]-(alkoxy en C₁ à C₄), alkoxyle en C₁ à C₄, un groupe de formule II 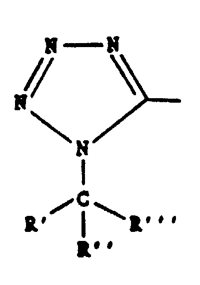 dans laquelle R' représente un groupe méthyle ou carboxy, R" représente l'hydrogène et R''' est choisi entre des groupes benzyle [facultativemenet substitué avec un, deux ou trois groupes choisis entre les groupes hydroxy, halogéno et phénoxy (facultativement substitué avec un, deux ou trois groupes choisis entre des groupes hydroxy et halogéno)], alkyle en C₁ à C₅, -(CH₂)ₙCO₂H, -CH₂SH, -CH₂SCH₃, imidazolinylméthylène, indolinylméthylène, CH₃CH (OH), CH₂OH, H₂N(CH₂)₄- (facultativement sous forme protégée) ou H₂NC(NH)NH(C-H₂)₃ (facultativement sous forme protégée) ; sous la réserve générale qu'au moins un de R₅, R₆, R₇ et R₈ soit choisi dans le groupe consistant en -CH=CH₂, -O-(CH₂)ₚOH, -O-(CH₂)ₚ-O-(CH₂)ₙpyridine-2-yle, -O-(CH₂)ₚ-O-(CH₂)ₙ-pyridine-3-yle, -O-(CH₂)ₚ-O-(CH₂)ₙpyridine-4-yle, -O-(CH₂)ₚ-O-(CH₂)ₙ-1-(alkyle en C₁ à C₄)-1H-5-tétrazole, -O-(CH₂)ₚ-O-(CH₂)ₙ-pyrimidine, -O-(CH₂)ₚ-O-(CH₂)ₙ-2-benzoxazole, -O-(CH₂)ₚ-O-(CH₂)ₙ-2-benzothiazole, -O-(CH₂)ₚ-O-(CH₂)ₙ-(alkyle en C₁ à C₄)-triazole, -O-(CH₂)ₚ-O-(CH₂)ₙ-(alkyle en C₁ à C₄)-imidazole, -O-(CH₂)ₚ-O-(CH₂)ₚ-OR₁₅, -O-(CH₂)ₚ-S-R₁₅, -O-(CH₂)ₚ-O-(CH₂)ₚNR₉R₁₀, -O-(CH₂)ₚ-S-(CH₂)ₚNR₉R₁₀, -O-(CH₂)ₚ-S-(CH₂)ₚ-OR₁₅, -O-(CH₂)ₚ-S(O)-R₁₅, -O-(CH₂)ₚ-S(O₂)-R₁₅, -O-(CH₂)ₚ-S(O)-(CH₂)ₚNR₉R₁₀, -O-(CH₂)ₚ-S(O)-(CH₂)ₚOR₁₅, -O-(CH₂)ₚ-S(O₂)-(CH₂)ₚNR₉R₁₀, -O-(CH₂)ₚ-S(O₂)-(CH₂)ₚ-OR₁₅, -O-(CH₂)ₚ-[4-[(CH₂)ₚOR₁₅]-1-pipérazine], -O-(CH₂)ₚ-[4-(CH) (phényl)₂-1-pipérazinel [le groupe phényle étant facultativement substitué avec un, 2 ou 3 groupes alkyle en C₁ à C₄, alkoxy en Cₗ à C₄, halogéno, OH, trifluorométhyle ou-CO₂ (alkyle en C₁ à C₄)], -O-(CH₂)ₚ-[4-(CH₂)_{q}phényl-1-pipérazine] [le groupe phényle étant facultativement substitué avec un, 2 ou 3 groupes alkyle en C₁ à C₄, alkoxy en C₁ à C₄, halogéno, OH, trifluorométhyle ou -CO₂(alkyle en C₁ à C₄)],-O-(CH₂)ₚ-[4-(CH₂)_{q}pyridinyl-1-pipérazinel [le groupe pyridinyle étant facultativement substitué avec un, 2 ou 3 groupes alkyle en C₁ à C₄, alkoxy en C₁ à C₄, halogéno, OH, trifluorométhyle, NR₉R₁₀ ou -CO₂(alkyle en C₁ à C₄)], -O-(CH₂)ₚ-[4-(pyridinyle à substituant NR₉R₁₀)-1-pipérazine, -O-(CH₂)ₚ-(1-pipéridine à substituant OH), -O-(CH₂)ₚ-1-pyrrolidine-2-one ;
R₁₅ est choisi entre H, des groupes alkyle en C₁ à C₅, -(CH₂)ₙphényle [le groupe phényle étant facultativement substitué avec un, 2 ou 3 groupes alkyle en C₁ à C₄, alkoxy en C₁ à C₄, halogéno, OH, trifluorométhyle ou -CO₂(alkyle en C₁ à C₄)],-(CH₂)ₙ-pyridine-1-yle, -(CH₂)ₙpyridine-2-yle, -(CH₂)ₙpyridine-3-yle, -(CH₂)ₙpyridine-4-yle, -(CH₂)ₙ-(1-(alkyle en C₁ à C₄)-1H-5-tétrazole, -(CH₂)ₙ-pyrimidine, -(CH₂)ₙ-2-benzoxazole, -(CH₂)ₙ-2-benzothiazole, -(CH₂)ₙ-(alkyle en C₁ à C₄)-triazole, -(CH₂)ₙ-(alkyle en C₁ à C₄)-imidazole ;
n a une valeur de 0 à 5 ;
p a une valeur de 2 à 5 ;
q a une valeur de 1 à 5 ;
et ses sels et hydrates pharmaceutiquement acceptables.

2. Composé suivant la revendication 1, dans lequel R₉ et R₁₀ sont pris conjointement avec N et forment un noyau amine hétérocyclique saturé ou insaturé choisi parmi les définitions (aa), (bb), (cc), (dd), (ee) et (ff) mentionnées dans la revendication 1.

3. Composé suivant la revendication 1, dans lequel Z représente H ou un groupe alkyle en C₁ à C₅.

4. Composé suivant la revendication 3, dans lequel n est égal à 0 ou 1 et R₉ et R₁₀, pris conjointement avec N, forment un groupe 4-morpholine.

5. Composé suivant la revendication 4, dans lequel Z représente H.

6. Composé suivant la revendication 5, dans lequel n est égal à 0.

7. Composé suivant la revendication 2, dans lequel au moins un membre choisi entre R₅, R₆, R₇ et R₈ est choisi entre :
-O-(CH₂)ₚ-S-R₁₅, -O-(CH₂)ₚ-[4-(CH)phényl)₂-1-pipérazine], -CH=CH₂, ou -O-(CH₂)ₚO-(CH₂)ₚOR₁₅.

8. Composé suivant la revendication 1, choisi dans le groupe consistant en les composés suivants :
Composé 217 2-(4-morpholinyl)-7-phénylméthoxy-8-vinyl-4H-1-benzopyranne-4-one;
Composé 219 8-méthyl-7-[(2-thiométhyl)éthyl]oxy-2-(4-morpholinyl)-4H-1-benzopyranne-4-one;
Composé 220 8-méthyl-2-(4-morpholinyl)-7-[2-(4-(2-hydroxy)éthyl-1-pipérazinyl)éthyl]oxy-4H-1-benzopyranne-4-one ;
Composé 222 7-[2-(hydroxy)éthyl]oxy-8-méthyl-2-(4-morpholinyl)-4H-1-benzopyranne-4-one;
Composé 223 8-méthyl-2-(4-morpholinyl)-7-[2-(2-thiopyridinyl)éthyl]oxy-4H-1-benzopyranne-4-one;
Composé 226 8-méthyl-7-[2-((1-méthyl-1,3-imidazole-2-yl)thio)éthyl]oxy-2-(4-morpholinyl)-4H-1-benzopyranne-4-one ;
Composé 228 8-méthyl-7-[2-((4-méthyl-1,2,4-triazole-3-yl)-thio)éthyl]oxy-2-(4-morpholinyl)-4H-1-benzopyranne-4-one ;
Composé 230 8-méthyl-7-[2-((1-méthyl-5-tétrazoyl)thio)éthyl]oxy-2-(4-morpholinyl)-4H-1-benzopyranne-4-one ;
Composé 231 8-méthyl-2-(4-morpholinyl)-7-[2-((2-pyrimidinyl)thio)éthyl]oxy-4H-1-benzopyranne-4-one;
Composé 234 7-[2-((2-(bis-N,N'-diéthylamino)éthyl)thio)éthyl]oxy-8-méthyl-2-(4-morpholinyl)-4H-1-benzopyranne-4-one ;
Composé 235 8-méthyl-7-[2-((2-benzoxazolyl)thio)éthyl]oxy-2-(4-morpholinyl)-4H-1-benzopyranne-4-one ;
Composé 236 8-méthyl-7-[2-((2-benzothioazolyl)thio)éthyl]-oxy-2-(4-morpholinyl)-4H-1-benzopyranne-4-one ;
Composé 237 7-[2-(4-(3-éthylamino-pyridine-2-yl)-1-pipérazinyl)éthyl]oxy-8-méthyl-2-(4-morpholinyl)-4H-1-benzopyranne-4-one ; et
Composé 238 8-méthyl-2-(4-morpholinyl)-7-[2-(pyrrolidinone-1-yl)éthyl]oxy-4H-1-benzopyranne-4-one ;
ou un de ses sels ou hydrates pharmaceutiquement acceptables.

9. Composé suivant la revendication 1, choisi dans le groupe consistant en les composés suivants :
Composé 242 2-(4-morpholinyl)-8-[2-(2-pyridinylthio)éthoxy]-4H-1-benzopyranne-4-one;
Composé 244 8-[2-[4-[3-(éthylamino)-2-pyridinyl]-1-pipérazinyl]éthoxy]-2-(4-morpholinyl)-4H-1-benzopyranne-4-one ;
Composé 246 8-méthyl-2-(4-morpholinyl)-7-[2-(phénylsulfinyl)éthoxy]-4H-1-benzopyranne-4-one;
Composé 253 7-[2-[(2-méthoxyphényl)thio]éthoxy]-8-méthyl-2-(4-morpholinyl)-4H-1-benzopyranne-4-one;
Composé 269 8-méthyl-2-(4-morpholinyl)-7-[2-(2-pipéridinyloxy)éthoxy]-4H-1-benzopyranne-4-one;
Composé 296 2-(4-morpholinyl)-8-[2-[4-(phénylméthyl)-1-pipérazinyl]éthoxyl-4H-1-benzopyranne-4-one;
Composé 326 8-éthényl-7-[2-(4-méthyl-1-pipérazinyl)éthoxy]-2-(4-morpholinyl)-4H-1-benzopyranne-4-one ;
Composé 327 8-éthényl-2-(4-morpholinyl)-7-[2-(1-pipéridinyl)éthoxyl-4H-1-benzopyranne-4-one;
Composé 328 8-éthényl-1-(4-morpholinyl)-7-[2-(4-phényl-1-pipéradinyl)éthoxyl-4H-1-benzopyranne-4-one;
Composé 329 8-éthényl-2-(4-morpholinyl)-7-[2-(1-pyrrolidinyl)éthoxy]-4H-1-benzopyranne-4-one;
Composé 330 8-éthényl-2-(4-morpholinyl)-7-[2-(4-thiomorpholinyl)éthoxy]-4H-1-benzopyranne-4-one;
Composé 331 (R)-8-éthényl-7-[2-[2-(hydroxyméthyl)-1-pyrrolidinyl)éthoxy]-2-(4-morpholinyl)-4H-1-benzopyranne-4-one ;
Composé 333 7-[2-(2-méthoxyéthoxy)éthoxy]-8-méthyl-2-(4-morpholinyl)-4H-1-benzopyranne-4-one ; et
Composé 335 8-méthyl-2-(4-morpholinyl)-7-[2-(phénylthio)éthoxy]-4H-1-benzopyranne-4-one;
ou un de ses sels ou hydrates pharmaceutiquement acceptables.

10. Composé, qui est le suivant :
Composé 227 7-[2-((bis-N,N'-(2-méthxoy)éthoxy)amino)éthyl]oxy-8-méthyl-2-(4-morpholinyl)-4H-1-benzopyranne-4-one ; ou
Composé 229 7-[2-(N-éthyl-N'-((2-hydroxy)éthyl)amino)éthyl]oxy-8-méthyl-2-(4-morpholinyl)-4H-1-benzopyranne-4-one ;
ou un de ses sels ou hydrates pharmaceutiquement acceptables.

11. 7-[2-(4-éthyl-1-pipérazinyl)éthyl]oxy-8-méthyl-2-(4-morpholinyl)-4H-1-benzopyranne-4-one ou un de ses sels pharmaceutiquement acceptables.

12. Procédé pour la préparation d'une composition pharmaceutique, qui comprend la formulation d'un composé suivant l'une quelconque des revendications 1 à 11 avec un support pharmaceutiquement acceptable.

13. Procédé pour la préparation d'un composé suivant l'une quelconque des revendications 1 à 11, qui comprend la réaction d'un ester d'acide salicylique de formule A 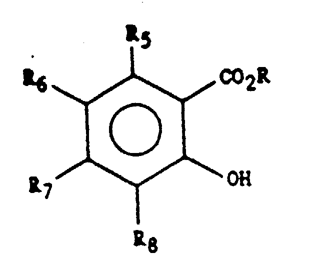 avec une ynamine de formule HC≡C-NR₉R₁₀.

14. Utilisation d'un composé suivant l'une quelconque des revendications 1 à 11, pour la production d'un médicament destiné à être utilisé dans la prévention ou le traitement de l'athérosclérose.

15. Utilisation suivant la revendication 4, dans laquelle R₉ et R₁₀ répondent aux définitions suivant la revendication 2.
